# EUROPEAN PATENT APPLICATION

(11) **EP 2 048 128 A1**
(43) Date of publication of application: **15.04.2009**
(21) Application number: 07768365.4
(22) Date of filing: 09.07.2007
(51) Int. Cl.: C07C 69/653, C07C 67/11, C07C 67/29, C07C 69/712, C08F 16/24, C08F 20/22, G03F 7/039, H01L 21/027

(54) **POLYMERIZABLE COMPOUND HAVING ADAMANTANE STRUCTURE, PROCESS FOR PRODUCTION OF THE SAME, AND RESIN COMPOSITION**

(30) Priority: 04.08.2006 JP 2006213364
(71) Applicant: Idemitsu Kosan Co., Ltd., Chiyoda-ku Tokyo 100-8321 (JP)
(72) Inventor: HATAKEYAMA, Naoyoshi, Ichihara-shi, Chiba 299-0193 (JP); ONO, Hidetoshi, Ichihara-shi, Chiba 299-0193 (JP); ITO, Katsuki, Ichihara-shi, Chiba 299-0193 (JP)
(74) Representative: Vossius & Partner
(86) International application number: PCT/JP2007/063641
(87) International publication number: WO 2008/015876

(57) **Abstract**

A polymerizable compound having a fluorinated substituent (Z) represented by the general formula (1), an adamantane structure and a polymerizable group (A) having the structure represented by the general formula (1), a production method thereof, and a photoresist composition, a thermocurable resin composition and a photocurable resin composition containing a polymer obtained using the polymerizable compound are provided. Use of the polymerizable compound with the adamantane structure and a resin composition thereof in the present invention provides in the field of photolithography the effect of preventing a liquid immersion medium from penetration and improving dry etching resistance in a liquid immersion exposure method as well as reducing adhesion to a mold and improving dry etching resistance in a nanoimprint method.

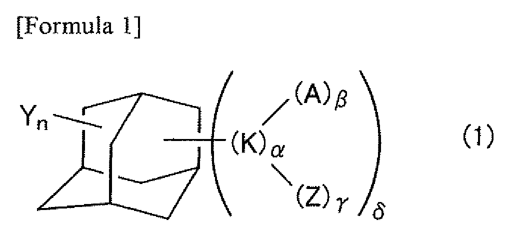

## Description

### Technical Field

The present invention relates to a polymerizable compound having the adamantane structure used as a material for photoresist lithography and nanoimprint lithography, a production method thereof, and a resin composition, in more detail, to a monomer for a functional resin such as a photosensitive resin and the like particularly in the field of photoresist lithography and nanoimprint lithography, a monomer having a fluorinated substituent, the adamantane structure and a polymerizable group useful as a raw material of a polymer using such a monomer, a production method thereof, and a polymer using the monomer, a photoresist composition, a thermocurable resin composition and a photocurable resin composition.

### Background Art

Adamantane has been known to be useful as a raw material for an ingredient of drugs, a highly functional industrial material and the like since it has high symmetry with a structure having four cyclohexane rings condensed in a cage form and is a stable compound and its derivative shows unique function. For example, it has been tried to use as an optical disc substrate, optical fiber or lens and the like since it has unique optical characteristics and heat resistance. (For example, see Patent Document 1 and Patent Document 2).
Adamantane esters have also been tried to use as a raw material of a resin for a photoresist by utilizing acid sensitivity, dry etching resistance, ultraviolet transparency and the like. (For example, see Patent Document 3).

On the other hand, liquid immersion exposure technology has been recently proposed as a technology to achieve the microfabrication associated with a demand for further finer fabrication in a photoresist lithography process. (For example, see Non-patent Document I). Such a liquid immersion exposure method is a technology to achieve high resolution by intervening a liquid immersion medium (a solvent such as water and the like) in the interface between a lens and a resist film. A nanoimprint lithography technology has also been proposed as a technology to achieve the microfabrication associated with a demand for further finer fabrication. (For example, see Patent Document 4). Such nanoimprint lithography is a technology to engrave a pattern by pressing a pattern-engraved form (mold) onto a composition containing a polymerizable compound, during which thermocuring or ultraviolet curing is performed.

In such a liquid immersion exposure method, such a problem has become apparent that the liquid immersion medium penetrates into the resist film, causing a development defect and a technique using a fluorine-containing compound has been proposed to prevent such penetration (for example, see Patent Document 5), but prevention of the penetration is still not sufficient and dry etching resistance in the subsequent production process is also not sufficient. In the nanoimprint method, a cured product adheres to the mold, causing such a problem as a development defect. While a technique using the fluorine-containing compound has been proposed to prevent the development defect, (see Patent Document 6), it is still not sufficient to prevent the development defect and also not sufficient in dry etching resistance in the subsequent production process.

Document 1: Japanese Patent Laid-Open Publication No. H06-305044
Document 2: Japanese Patent Laid-Open Publication No. H09-302077
Document 3: Japanese Patent Laid-Open Publication No. H04-39665
Document 4: Japanese Patent Laid-Open Publication No. 2003-100609
Document 5: Japanese Patent Laid-Open Publication No. 2005-284238
Document 6: Japanese Patent Laid-Open Publication No. 2002-184719
Non-patent Document 1: Proceeding of SPIE (published in USA), 2002, Vol. 4691, p. 459-465.

### Disclosure of the Invention

### Problems to be Solved by the Invention

An object of the present invention is to provide, under such a condition, a new polymerizable compound having the adamantane structure useful as a monomer for a functional resin such as a photosensitive resin and the like in a field of photoresist lithography, a production method thereof and a resin composition in order to solve such problems as penetration of the liquid immersion medium and insufficient resistance in dry etching in the liquid immersion exposure method as well as such problems as adhesion to a mold and insufficient resistance in dry etching in the nanoimprint method.

### Means for Solving the Problems

The present inventors have earnestly studied to achieve the above object and consequently found a polymerizable compound with a particular structure having a fluorinated substituent with a particular structure, an adamantane structure and a polymerizable group is a new compound suitable to its object and such a compound can be efficiently produced by reacting a polymerizable compound having both the corresponding adamantane structure and the polymerizable group with a compound having a fluorinated substituent or reacting a compound having both the adamantane structure and the fluorinated substituent with a polymerizable compound having the polymerizable group. The present invention has been completed based on such a teaching.

That is, the present invention provides a following polymerizable compound having the adamantane structure, a resin composition and a production method thereof. 1. A polymerizable compound having the adamantane structure and a structure represented by the general formula (1).

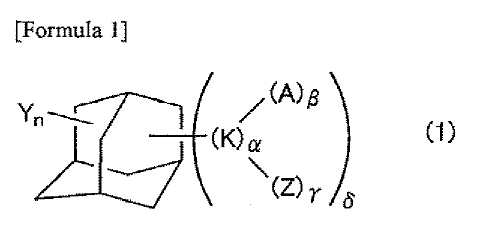

(In the formula, A is a group containing a polymerizable group represented by the formulas (2) or (3) and a plurality of A may be identical or different. K is a linkage group represented by any one of the formulas (4) to (8) and a plurality of K may be identical or different. Z is the following fluorinated substituent and a plurality of Z may be identical or different. Y is a substituent on adamantane and represents a hydrogen atom, an alkyl group having 1 to 10 carbon atoms, a halogen atom, a hydroxyl, mercapto or methylcyano group or =O or =S formed by joining two Ys. A plurality of Y may be identical or different. α is an integer of 1 or more and β and γ each are an integer of 0 or more, but one or more of A and Z are included in the general formula (1). δ is an integer of 1 to 16, n is an integer of 0 to 15 and δ + n is equal to 16.

A (Polymerizable group):

[Formula 2] CH₂=CR^{o}- (2) CH≡C- (3)

R⁰ represents a hydrogen atom, a fluorine atom or a methyl, ethyl or trifluoromethyl group.

K (Linkage group):

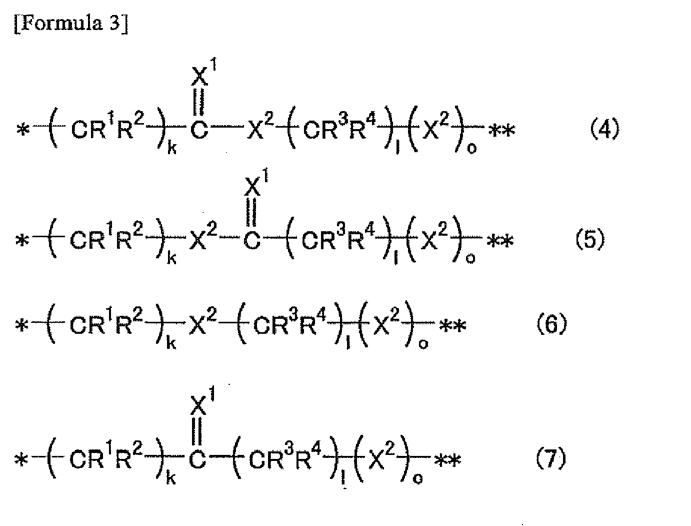

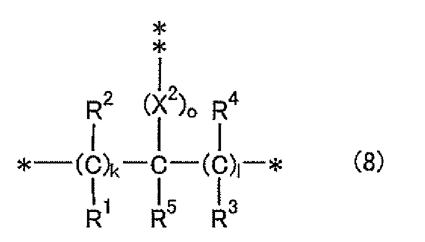

R¹, R², R³, R⁴ and R⁵ each independently represent a hydrogen atom, an alkyl group having 1 to 10 carbon atoms optionally containing a heteroatom, or a halogen atom, k and 1 each represent an integer of 0 to 10, X¹ and X² each independently represent an oxygen atom, a sulfur atom or an NR' group. R¹ is a hydrogen atom or an alkyl group having 1 to 10 carbon atoms optionally containing a heteroatom, single asterisk (*) represents the side of a (polymerizable group or the side of an end group, a double asterisk (**) represents the side of an adamantane ring and o represents 0 or 1.

Z (Fluorinated substituent):
Z represents an alkyl group having 1 to 30 carbon atoms or a cycloalkyl group having 5 to 30 carbon atoms, has to include a fluorine atom in part of the structure thereof, and optionally contains in part of the structure thereof at least one kind selected from a heteroatom or hydroxyl, mercapto, ether, thioether, cyano, ketone, thioketone, ketal, thioketal, acetal, thioacetal, lactone, thiolactone, carbonate, thiocarbonate, amine, amide, alkylsulfonyl, ester and thioester groups.)

2. The polymerizable compound having the adamantane structure described above in 1,having the structure represented by the general formula (9). 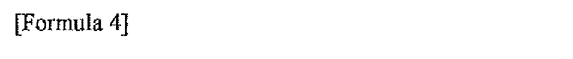 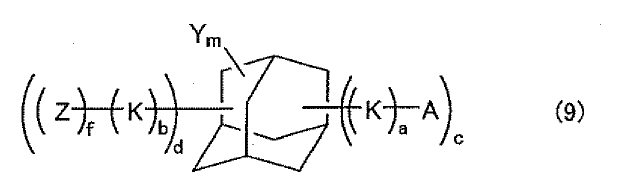 (A (polymerizable group), K (linkage group), Z (fluorinated substituent) and Y (substituent on adamantane) are similar to those in the general formula (1), a, b, c, d and f each are an integer of 1 or more, m is an integer of 0 to 14, and c + d+ m is equal to 16).

3. The polymerizable compound having the adamantane structure described above in 2, having the structure represented by the general formula (10).

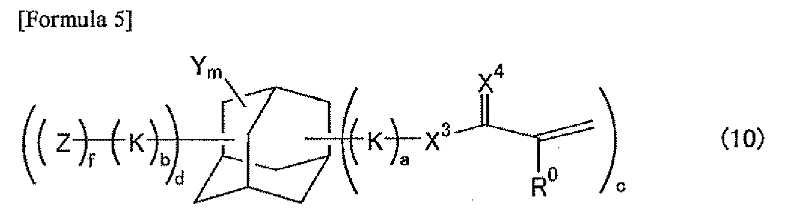

(K (linkage group), Z (fluorinated substituent) and Y (substituent on adamantane) are similar to those in the general formula (1), R⁰ is similar to that in the general formula (2) and a, b, c, d, f, and m are similar to those in the general formula (9). X³ and X⁴ each independently represent an oxygen atom, a sulfur atom or a NR' group. R' is a hydrogen atom or an alkyl group having to 10 carbon atoms optionally containing a heteroatom.)

4. The polymerizable compound having the adamantane structure described above in 2, having the structure represented by the general formula (11).

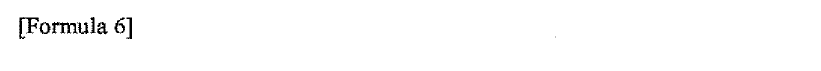

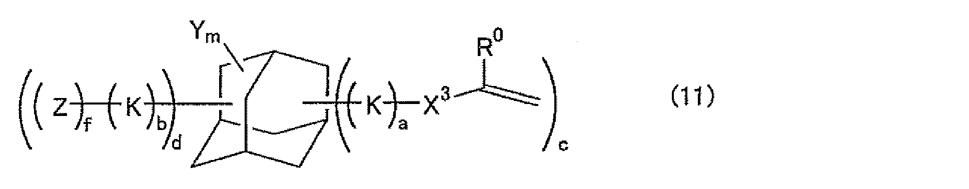

(K (linkage group), Z (fluorinated substituent), Y (substituent on adamantane), R⁰, X³, a, b, c, d, f, and m are similar to those in the general formula (10).)

5. The polymerizable compound having the adamantane structure described above in 3, having the structure represented by the general formula (12). 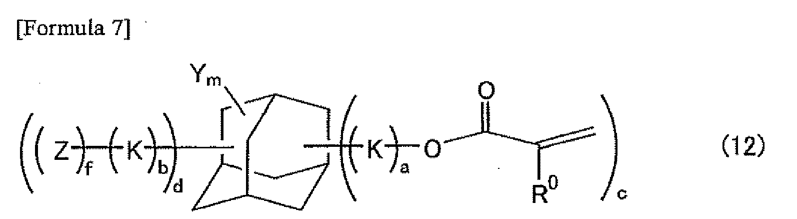 (K (linkage group), Z (fluorinated substituent), Y (substituent on adamantane), R⁰, a, b, c, d, f, and m are similar to those in the general formula (10).)

6. The polymerizable compound having the adamantane structure described above in 4, having the structure represented by the general formula (13). 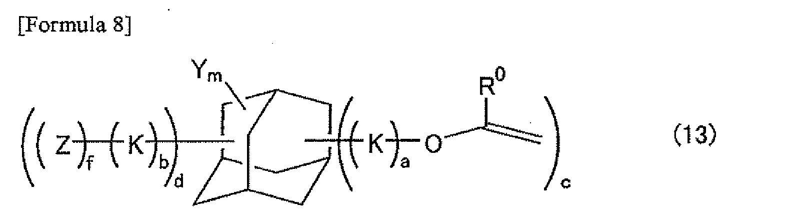 (K (linkage group), Z (fluorinated substituent), Y (substituent on adamantane), R⁰, a, b, c, d, f, and m are similar to those in the general formula (10).)

7. The polymerizable compound having the adamantane structure described above in 2, having the structure represented by the general formula (14).

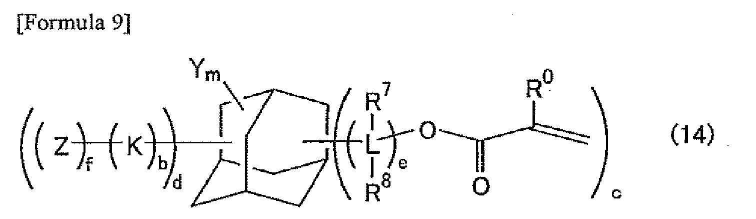

(K (linkage group), Z (fluorinated substituent), Y (substituent on adamantane), R⁰, b, c, d, f, and m are similar to those in the general formula (10) and R⁷ and R⁸ each independently represent a hydrogen atom, an alkyl group having 1 to 10 carbon atoms optionally containing a heteroatom or a halogen atom. L independently represents a carbon, oxygen, nitrogen or sulfur atom. A plurality of R⁷ and a plurality of R⁸ each may be identical or different. However, when L is an oxygen, nitrogen or sulfur atom, either one or both of R⁷ and R⁸ are absent. e is an integer of 0 to 5.)

8. The polymerizable compound having the adamantane structure described above in 2, having the structure represented by the general formula (15).

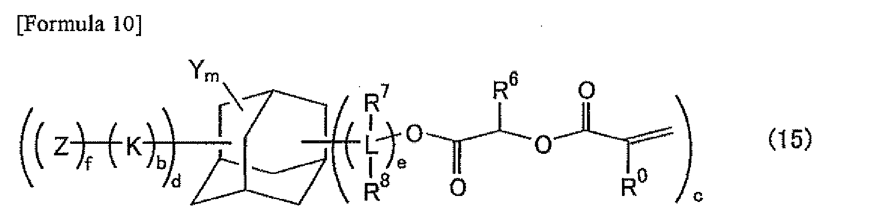

(K (linkage group), Z (fluorinated substituent),Y (substituent on adamantane), L, R⁰, R⁷, R⁸, b, c, d, e, f, and m are similar to those in the general formula (14). R⁶ represents a hydrogen atom, an alkyl group having 1 to 10 carbon atoms optionally containing a heteroatom or a halogen atom.)

9. The polymerizable compound having the adamantane structure described above in 2, having the structure represented by the general formula (16).

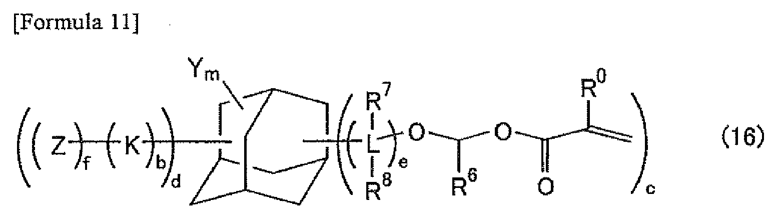

(K (linkage group), Z (fluorinated substituent), Y (substituent on adamantane), L, R⁰, R⁶, R⁷, R⁸, b, c, d, e, f, and m are similar to those in the general formula (15).)

10. The (polymerizable compound having the adamantane structure described above in 2, having the structure represented by the general formula (17).

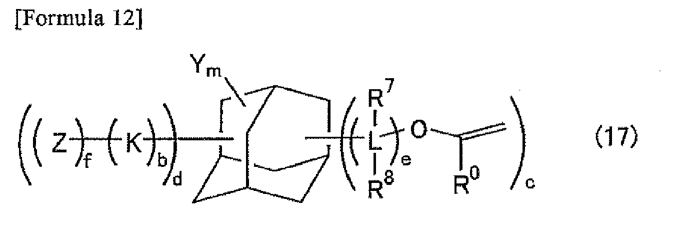

(K (linkage group), Z (fluorinated substituent),Y (substituent on adamantane), L, R⁰, R⁷, R⁸, b, c, d, e, f, and m are similar to those in the general formula (15).)

11. The polymerizable compound having the adamantane structure described above in 2, having the structure represented by the general formula (18).

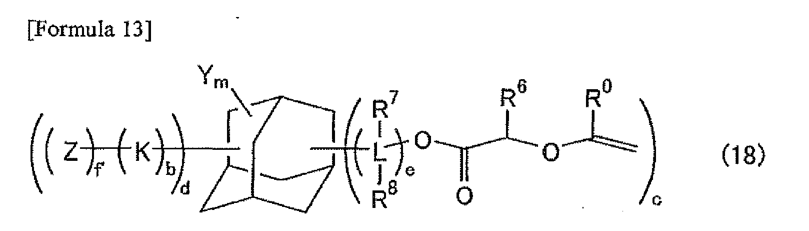

(K (linkage group), Z (fluorinated substituent), Y (substituent on adamantane), L, R⁰, R⁶, R⁷, R⁸, b, c, d, f, and m are similar to those in the general formula (15).)

12. The polymerizable compound having the adamantane structure described above in 2, having the structure represented by the general formula (19),

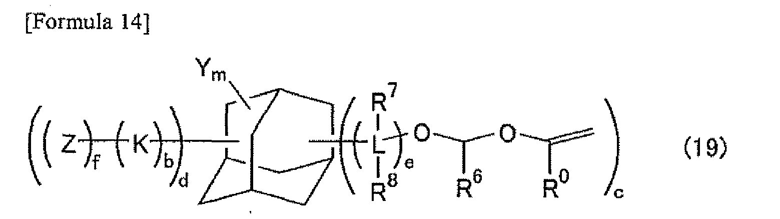

(K (linkage group), Z (fluorinated substituent), Y (substituent on adamantane), L, R⁰, R⁶, R⁷, R⁸, b, c, d, f, and m are similar to those in the general formula (15).)

13. The polymerizable compound having the adamantane structure described above in 2, in which at least one of R⁹ to R¹¹ in the general formula (20) is represented by any one of the general formula (21), the general formula (22), the general formula (23), the general formula (24), the general formula (25), and the general formula (26).

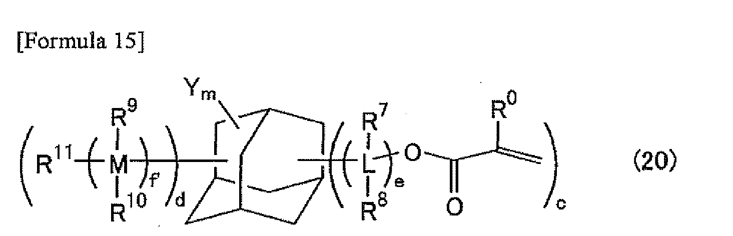

(Y (substituent on adamantane), R⁰, c, d, and m are similar to those in the general formula (10) and L and M each independently represent a carbon, oxygen, nitrogen or sulfur atom. R⁷, R⁸, R⁹, R¹⁰, and R¹¹ each independently represent a hydrogen atom, an alkyl group having 1 to 10 carbon atoms optionally containing a heteroatom, a halogen atom or =O or =S formed by joining two of R⁷, R⁸ and R⁹ to R¹¹. A plurality of R⁷, a plurality of R⁸, a plurality of R⁹, a plurality of R¹⁰ and R¹¹ each may be identical or different. However, when L and M are an oxygen, nitrogen or sulfur atom, either one or both of R⁷ and R⁸ in L are absent and either one or both of R⁹ and R¹⁰ in M are absent. e and f' each are an integer of 0 to 5.)

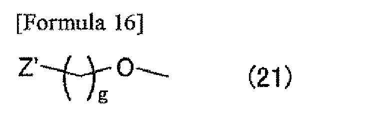

(Z' (fluorinated substituent) represents an alkyl group having 1 to 30 carbon atoms or a cycloalkyl group having 5 to 30 carbon atoms, in which the hydrogen atom in the structure is fully replaced by fluorine atoms and optionally contains in part of the structure thereof at least one kind from a heteroatom, ether, thioether, cyano, ketone, thioketone, katal, thioketal, acetal, thioacetal, lactone, thiolactone, carbonate, thiocarbonate, ester and thioester groups. g is an integer of 0 or more.)

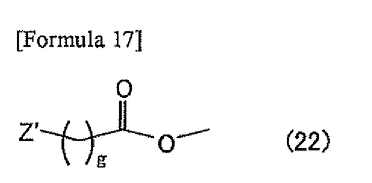

(Z' (fluorinated substituent) and g are similar to those in the general formula (21).)

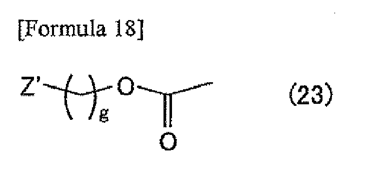

(Z' (fluorinated substituent) and g are similar to those in the general formula (21).)

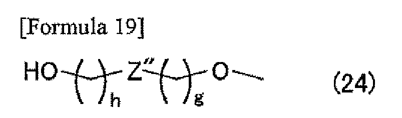

(Z" (fluorinated substituent) represents an alkylene group having 1 to 30 carbon atoms or a cycloalkylene group having 5 to 30 carbon atoms, in which the hydrogen atom in the structure is fully replaced by fluorine atoms and optionally contains in part of the structure thereof at least one kind selected from a heteroatom, ether, thioether, cyano, ketone, thioketone, ketal, thioketal, acetal, thioacetal, lactone, thiolactone, carbonate, thiocarbonate, ester and thioester groups, h and g are an integer of 0 or more.)

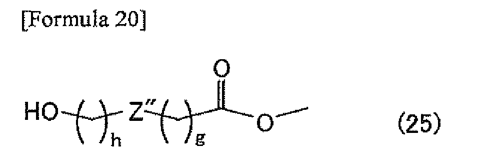

(Z" (fluorinated substituent), g, and h are similar to those in the general formula (24).)

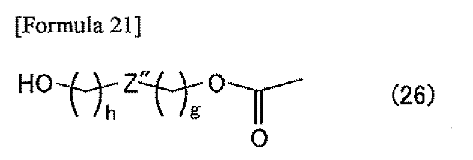

(Z" (fluorinated substituent), g, and h are similar to those in the general formula (24).)

14. The polymerizable compound having the adamantane structure described above in 2, in which at least one of R⁹ to R¹¹ in the general formula (27) is represented by any one of the general formula (28), the general formula (29), the general formula (30), the general formula (31), the general formula (32), and the general formula (33).

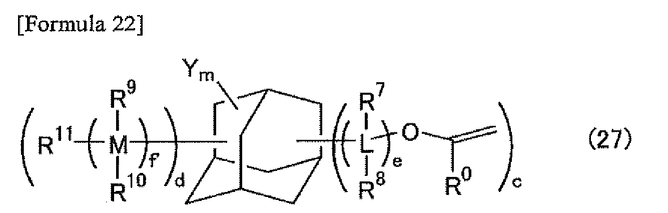

(Y (substituent on adamantane), L, M, R⁰, R⁷, R⁸, R⁹, R¹⁰, R¹¹, c, d, e, and f' are similar to those in the general formula (20).)

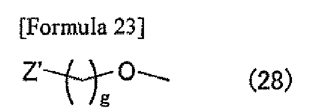

(Z' (fluorinated substituent) and g are similar to those in the general formula (21).)

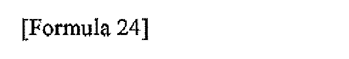

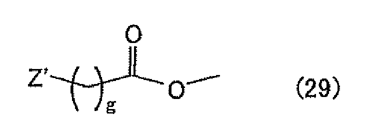

(Z' (fluorinated substituent) and g are similar to those in the general formula (21).)

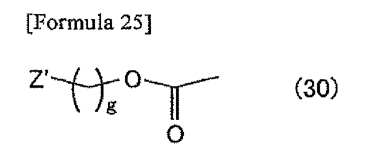

(Z' (fluorinated substituent) and g are similar to those in the general formula (21).)

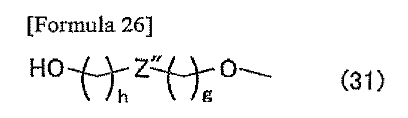

(Z" (fluorinated substituent), g, and h are similar to those in the general formula (24).)

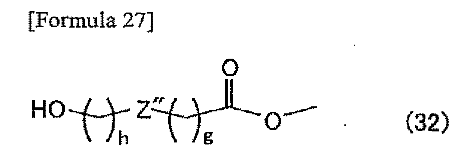

(Z" (fluorinated substituent), h, and g are similar to those in the general formula (24).)

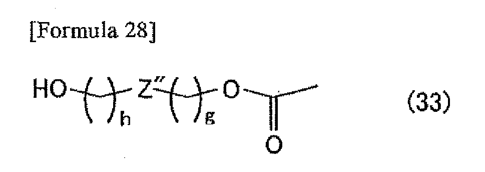

(Z" (fluorinated substituent), h, and g are similar to those in the general formula (24).)

15. A production method of the polymerizable compound having the adamantane structure, having the structure represented by the general formula (37) **characterized in that** a compound of the general formula (34), in which at least one of R⁹, R¹⁰, and R¹² is represented by the general formula (35) is reacted with a compound represented by the general formula (36).

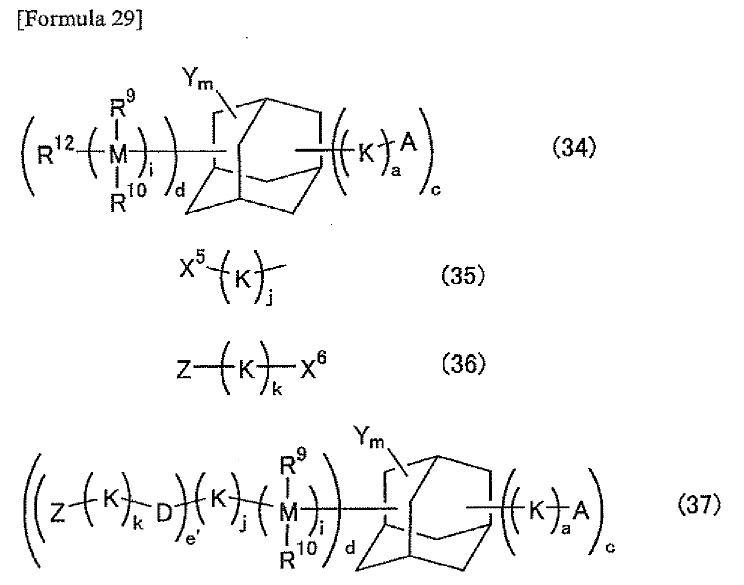

(A (polymerizable group), K (linkage group), Z (fluorinated substituent) ,Y (substituent on adamantane), a, c, d, and m are similar to those in the general formula (9), e' is an integer of 1 or more, i is an integer of 0 to 5, and j and k each are an integer of 0 or more. R⁹, R¹⁰, and R¹² each independently represent a hydrogen atom, an alkyl group having 1 to 10 carbon atoms optionally containing a heteroatom, a halogen atom or =O or =S formed by joining two of R⁹, R¹⁰ and R¹¹. A plurality of R⁹, a plurality of R¹⁰, a plurality of R¹² each may be identical or different. M independently represents a carbon, oxygen, nitrogen or sulfur atom. However, when M is an oxygen, nitrogen or sulfur atom, either one or both of R⁹ and R¹⁰ are absent. X⁵ and X⁶ are a reactive group, and when X⁵ is selected from a hydrogen atom, a halogen atom, an alkylsulfonyloxy, perfluoroalkylsulfonyloxy or alkyl-substituted phenylsulfonyloxy group, X⁶ represents a hydrogen atom or a group selected from a hydroxyl, mercapto, or amino group or salt thereof, whereas when X⁵ is selected from a hydrogen atom, a hydroxyl, mercapto or amino group or salt thereof, X⁶ represents a hydrogen atom, a halogen atom or a group selected from an alkylsulfonyloxy, perfluoroalkylsulfonyloxy or alkyl-substituted phenylsulfonyloxy group. D represents a linkage group formed by the reaction of X⁵ with X⁶.)

16. A production method of a polymerizable compound having the adamantane structure, having the structure represented by the general formula (40) **characterized in that** a compound represented by the general formula (3 8) is reacted with a compound represented by the general formula (39).

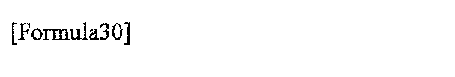

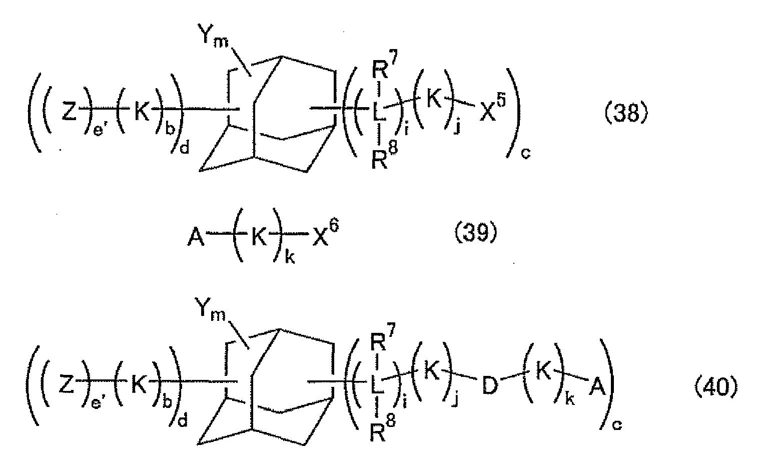

(A (polymerizable group), K (linkage group), Z (fluorinated substituent), Y (substituent on adamantane), X⁵, X⁶, D, c, d, e', i, j, k, and m are similar to those in the general formula (34) to (37) and L, R⁷ and R⁸ are similar to those in the general formula (14).)

17. A polymer having any one of the polymerizable compounds having the adamantane structure described above in 1 to 14 as a constituent.
18. A photoresist composition containing a polymer having any one of the polymerizable compound having the adamantane structure described above in 1 to 14 as a constituent.
19. A thermocurable resin composition containing any one of the polymerizable compounds having the adamantane structure described above in 1 to 14 as a constituent.
20. A photocurable resin composition containing any one of the polymerizable compounds having the adamantane structure described above in 1 to 14 as a constituent.

### Effect of the Invention

The polymerizable compound having the adamantane structure in the present invention is a polymerizable compound having a fluorinated substituent, the adamantane structure and a polymerizable group and represents a new monomer, in which the structure having the fluorinated substituent increases repellency of a liquid immersion medium (particularly water repellency) and mold releasability and the structure having the adamantane increases dry etching resistance, and a polymer and a composition containing it is provided by using it.
That is, use of the polymerizable compound having the adamantane structure in the present invention and a resin composition thereof has in the field of photolithography the effect of preventing the liquid immersion medium from penetration and improving dry etching resistance in the liquid immersion exposure method as well as reducing adhesion to a mold and improving dry etching resistance in the nanoimprint method, allowing favorable use thereof in this field.

### Brief Description of Drawings

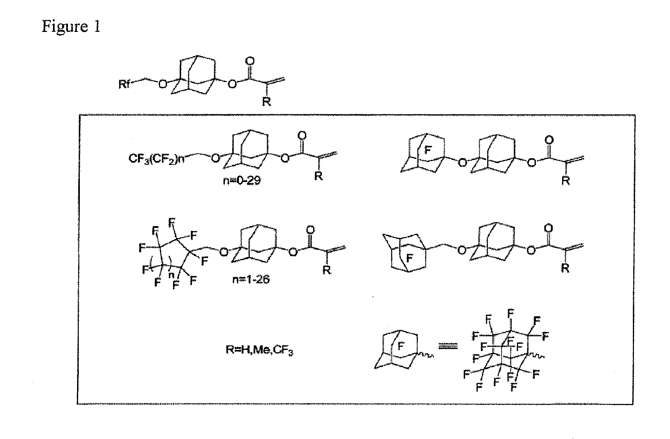

Fig. 1 is a constitutional formula illustrating a specific example of a polymerizable compound having the adamantane structure having a linkage of a fluorinated substituent of the general formula (21) in the polymerizable compound having the adamantane structure represented by the general formula (20).

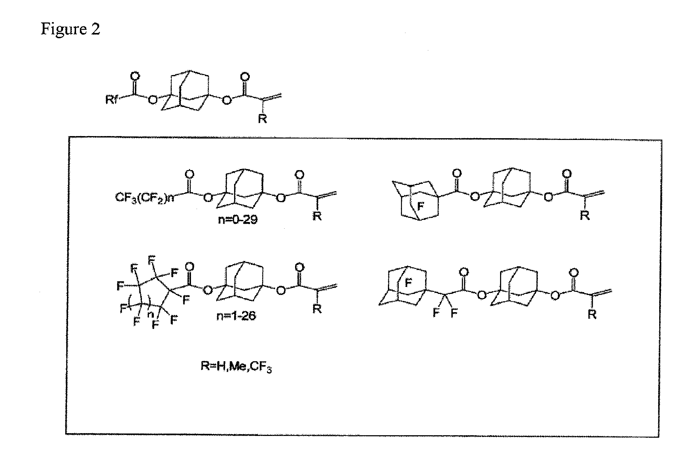

Fig. 2 is a constitutional formula illustrating a specific example (1) of a polymerizable compound having the adamantane structure having a linkage of a fluorinated substituent of the general formula (22) in the polymerizable compound having the adamantane structure represented by the general formula (20).

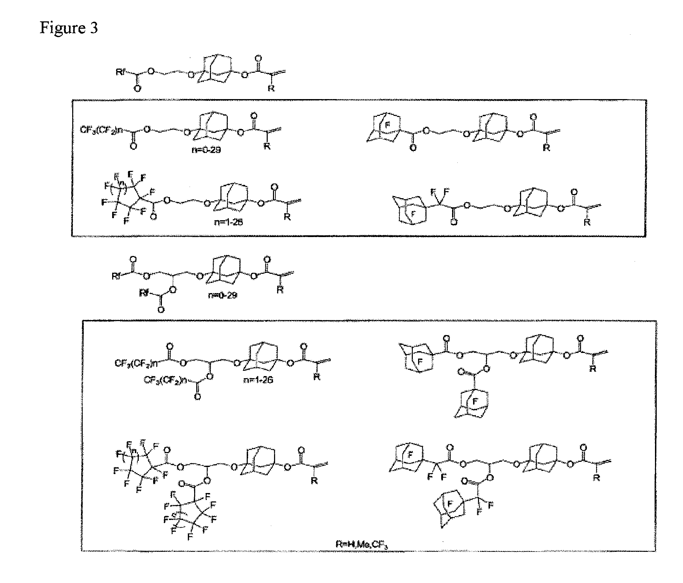

Fig. 3 is a constitutional formula illustrating a specific example (2) of a polymerizable compound having the adamantane structure having a linkage of a fluorinated substituent of the general formula (22) in the polymerizable compound having the adamantane structure represented by the general formula (20).

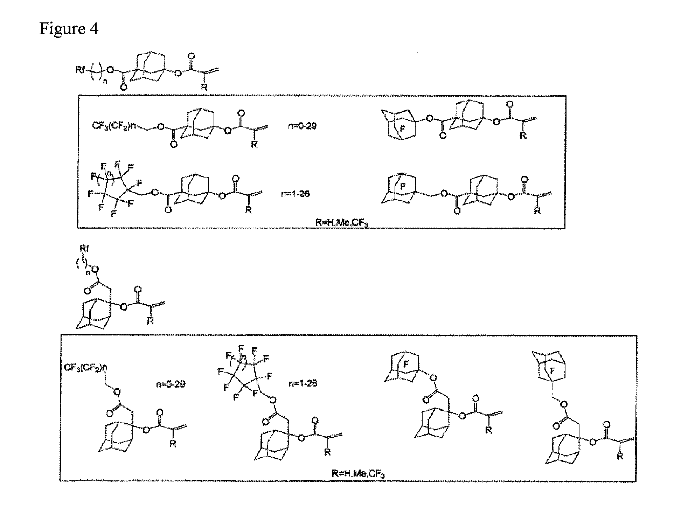

Fig. 4 is a constitutional formula illustrating a specific example (1) of a polymerizable compound having the adamantane structure having a linkage of a fluorinated substituent of the general formula (23) in the polymerizable compound having the adamantane structure represented by the general formula (20).

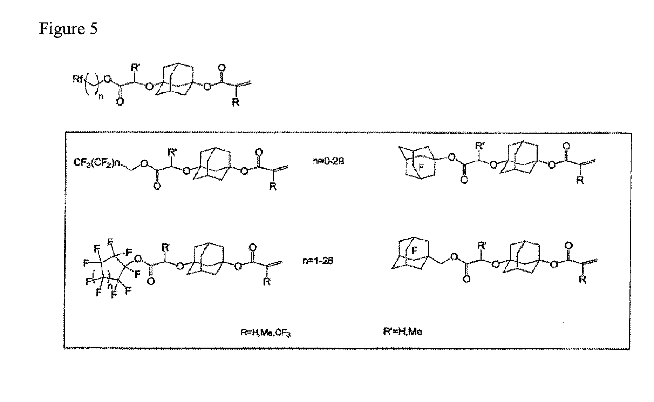

Fig. 5 is a constitutional formula illustrating a specific example (2) of a polymerizable compound having the adamantane structure having a linkage of a fluorinated substituent of the general formula (23) in the polymerizable compound having the adamantane structure represented by the general formula (20).

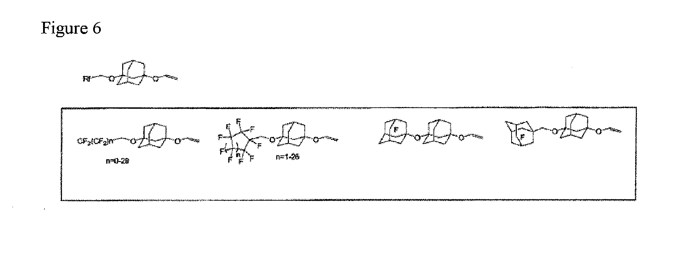

Fig. 6 is a constitutional formula illustrating a specific example of a polymerizable compound having the adamantane structure having a linkage of a fluorinated substituent of the general formula (28) in the polymerizable compound having the adamantane structure represented by the general formula (27).

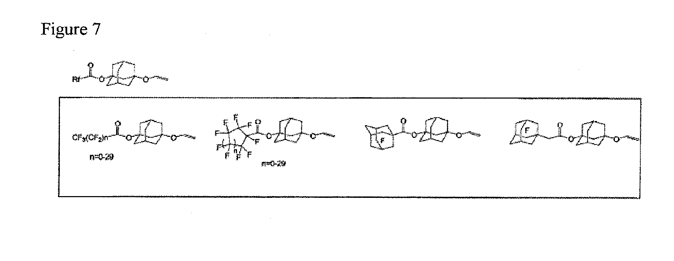

Fig. 7 is a constitutional formula illustrating a specific example (1) of a polymerizable compound having the adamantane structure having a linkage of a fluorinated substituent of the general formula (29) in the polymerizable compound having the adamantane structure represented by the general formula (27).

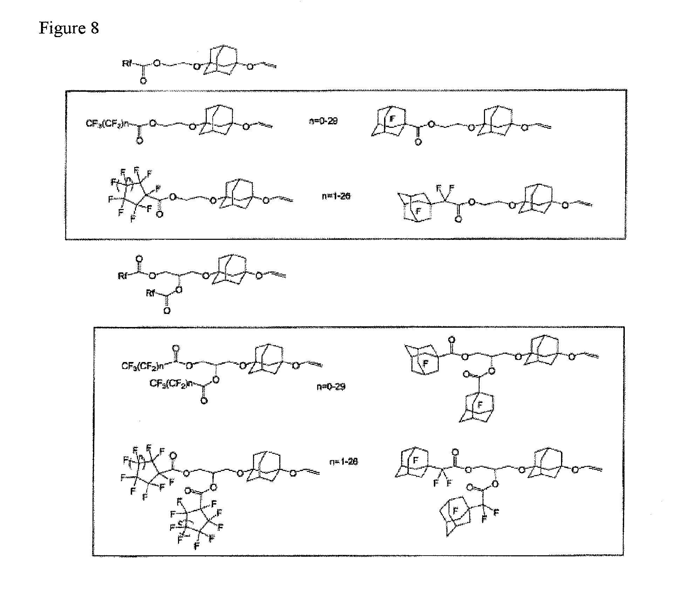

Fig. 8 is a constitutional formula illustrating a specific example (2) of a polymerizable compound having the adamantane structure having a linkage of a fluorinated substituent of the general formula (29) in the polymerizable compound having the adamantane structure represented by the general formula (27).

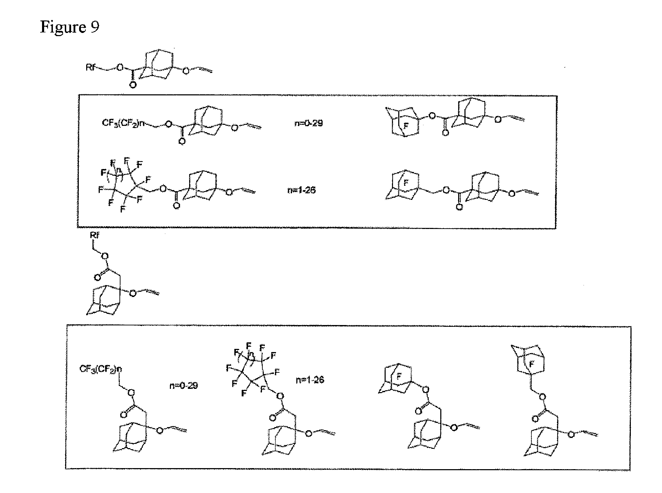

Fig. 9 is a constitutional formula illustrating a specific example (1) of a polymerizable compound having the adamantane structure having a linkage of a fluorinated substituent of the general formula (30) in the polymerizable compound having the adamantane structure represented by the general formula (27).

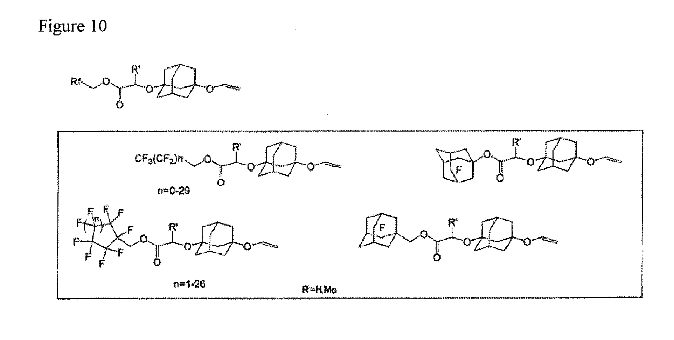

Fig. 10 is a constitutional formula illustrating a specific example (2) of a polymerizable compound having the adamantane structure having a linkage of a fluorinated substituent of the general formula (30) in the polymerizable compound having the adamantane structure represented by the general formula (27).

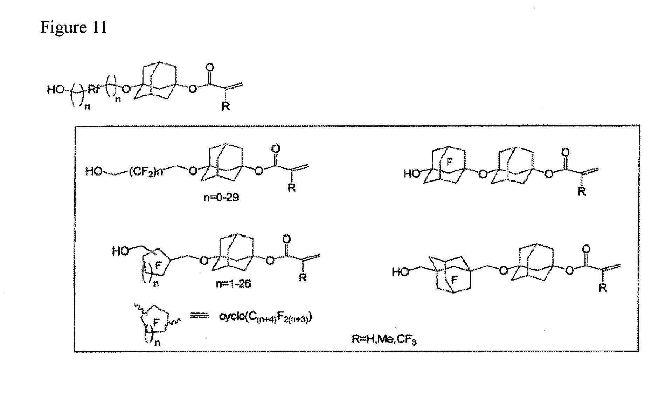

Fig. 11 is a constitutional formula illustrating a specific example of a polymerizable compound having the adamantane structure having a linkage of a fluorinated substituent of the general formula (24) in the polymerizable compound having the adamantane structure represented by the general formula (20).

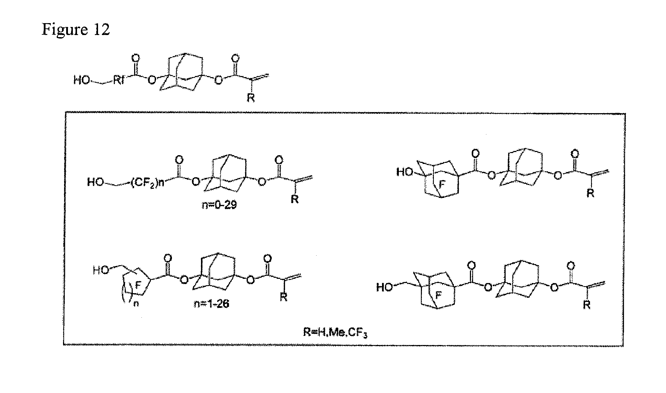

Fig. 12 is a constitutional formula illustrating a specific example (1) of a polymerizable compound having the adamantane structure having a linkage of a fluorinated substituent of the general formula (25) in the polymerizable compound having the adamantane structure represented by the general formula (20).

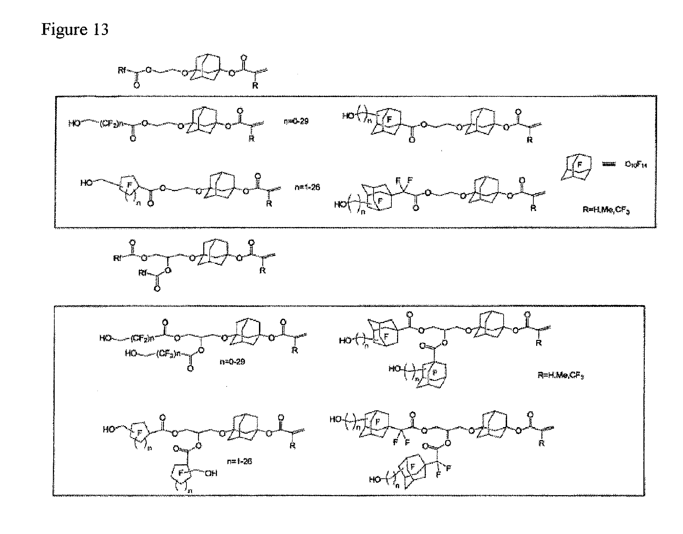

Fig. 13 is a constitutional formula illustrating a specific example (2) of a polymerizable compound having the adamantane structure having a linkage of a fluorinated substituent of the general formula (25) in the polymerizable compound having the adamantane structure represented by the general formula (20).

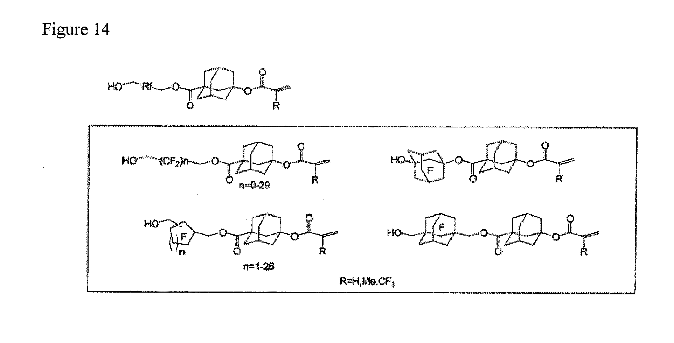

Fig. 14 is a constitutional formula illustrating a specific example (1) of a polymerizable compound having the adamantane structure having a linkage of a fluorinated substituent of the general formula (26) in the polymerizable compound having the adamantane structure represented by the general formula (20).

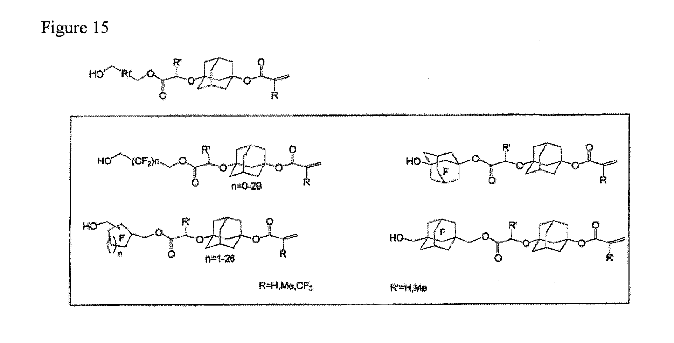

Fig. 15 is a constitutional formula illustrating a specific example (2) of a polymerizable compound having the adamantane structure having a linkage of a fluorinated substituent of the general formula (26) in the polymerizable compound having the adamantane structure represented by the general formula (20).

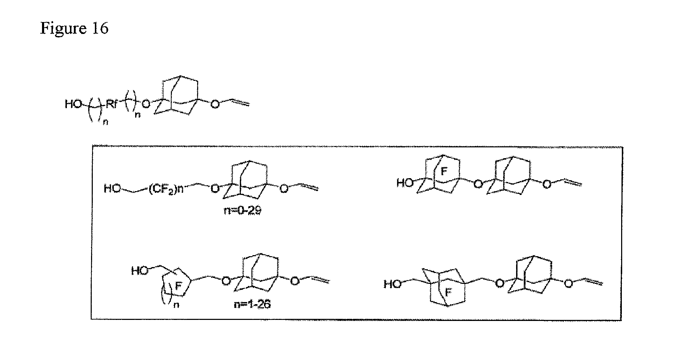

Fig. 16 is a constitutional formula illustrating a specific example of a polymerizable compound having the adamantane structure having a linkage of a fluorinated substituent of the general formula (31) in the polymerizable compound having the adamantane structure represented by the general formula (27).

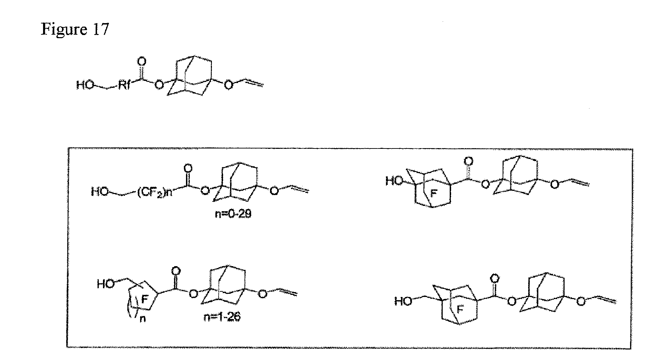

Fig. 17 is a constitutional formula illustrating a specific example (1) of a polymerizable compound having the adamantane structure having a linkage of a fluorinated substituent of the general formula (32) in the polymerizable compound having the adamantane structure represented by the general formula (27).

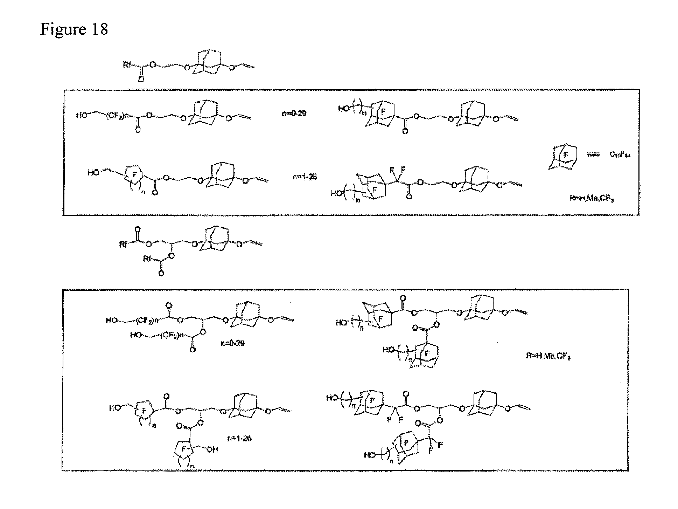

Fig. 18 is a constitutional formula illustrating a specific example (2) of a polymerizable compound having the adamantane structure having a linkage of a fluorinated substituent of the general formula (32) in the polymerizable compound having the adamantane structure represented by the general formula (27).

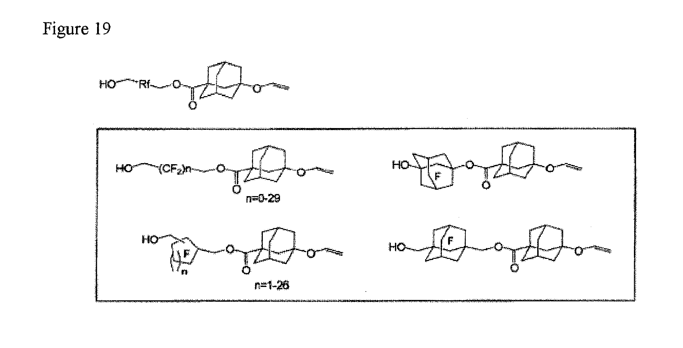

Fig. 19 is a constitutional formula illustrating a specific example (1) of a polymerizable compound having the adamantane structure having a linkage of a fluorinated substituent of the general formula (33) in the polymerizable compound having the adamantane structure represented by the general formula (27).

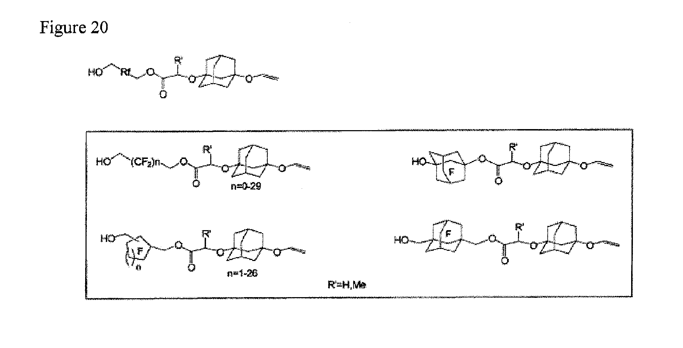

Fig. 20 is a constitutional formula illustrating a specific example (2) of a polymerizable compound having the adamantane structure having a linkage of a fluorinated substituent of the general formula (33) in the polymerizable compound having the adamantane structure represented by the general formula (27).

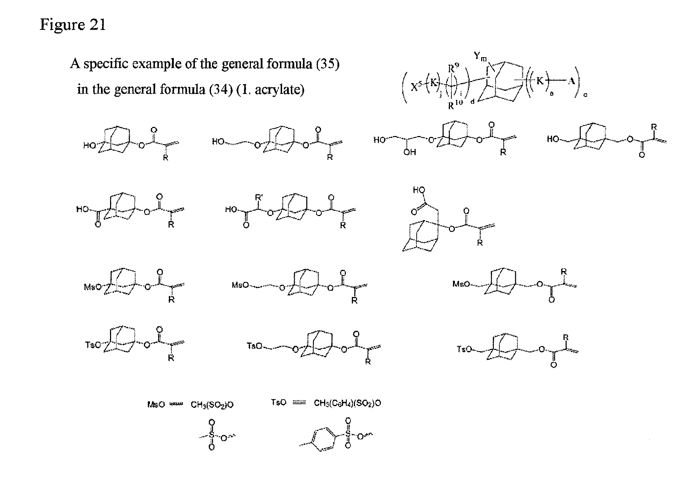

Fig. 21 is a constitutional formula illustrating a specific example of a compound (1. acrylate) in the general formula (34), in which at least one of R⁹, R¹⁰, and R¹² is represented by the general formula (35).

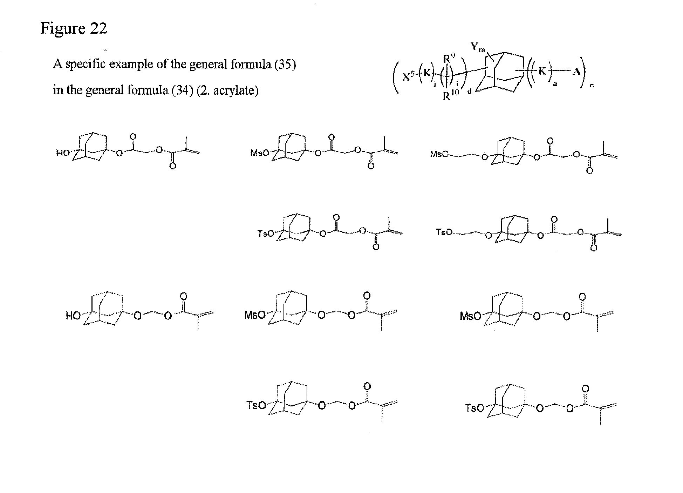

Fig. 22 is a constitutional formula illustrating a specific example of a compound (2. acrylate) in the general formula (34), in which at least one of R⁹, R¹⁰, and R¹² is represented by the general formula (35).

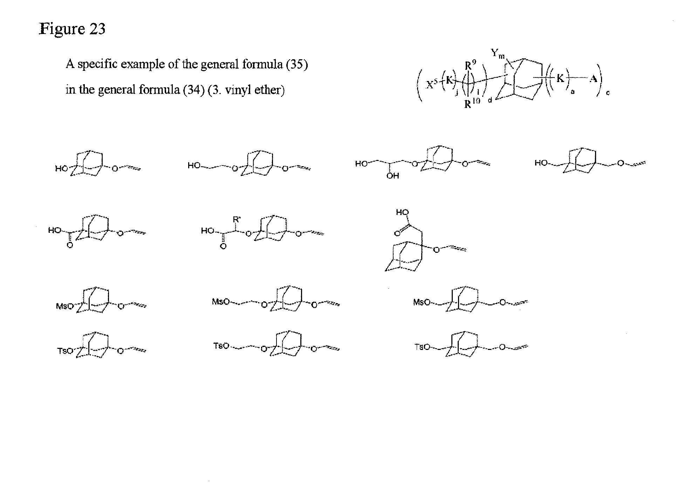

Fig. 23 is a constitutional formula illustrating a specific example of a compound (3. vinyl ether) in the general formula (34), in which at least one of R⁹, R¹⁰, and R¹² is represented by the general formula (35).

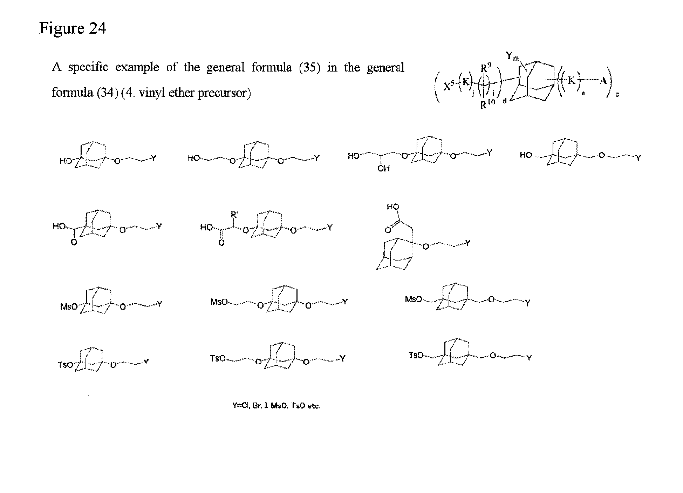

Fig. 24 is a constitutional formula illustrating a specific example of a compound (4. vinyl ether precursor) in the general formula (34), in which at least one of R⁹, R¹⁰,and R¹² is represented by the general formula (35).

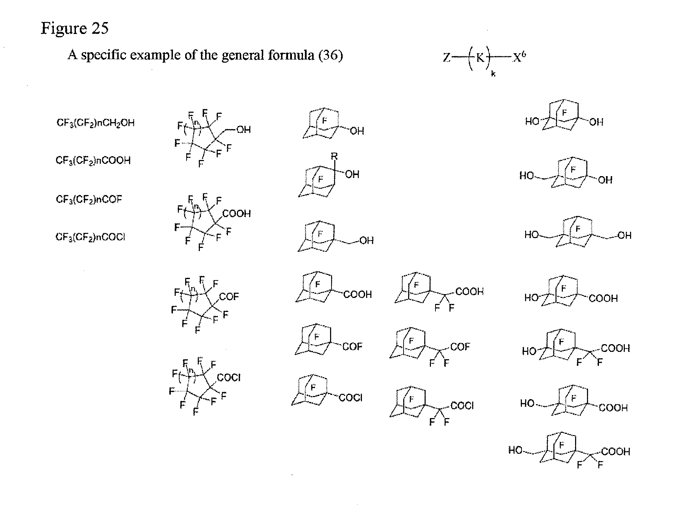

Fig. 25 is a constitutional formula illustrating a specific example of a compound represented by the general formula (36).

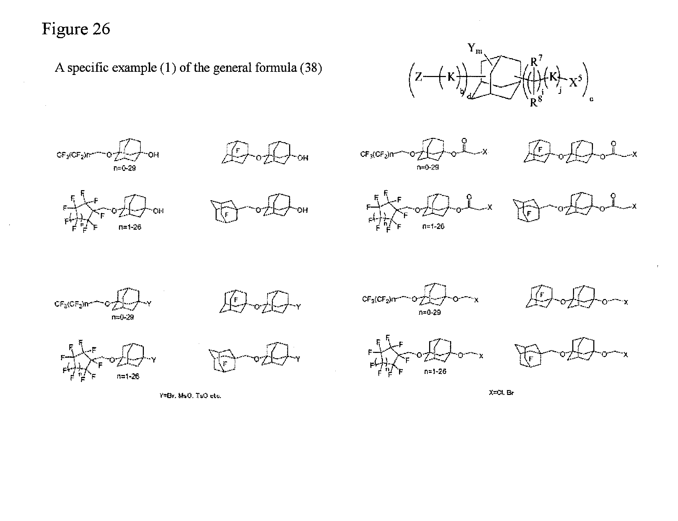

Fig. 26 is a constitutional formula illustrating a specific example (1) of a compound represented by the general formula (38).

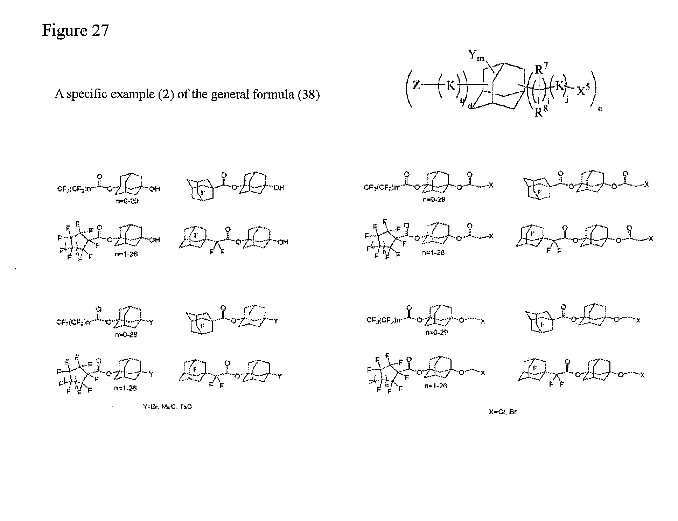

Fig. 27 is a constitutional formula illustrating a specific example (2) of a compound represented by a general formula (38).

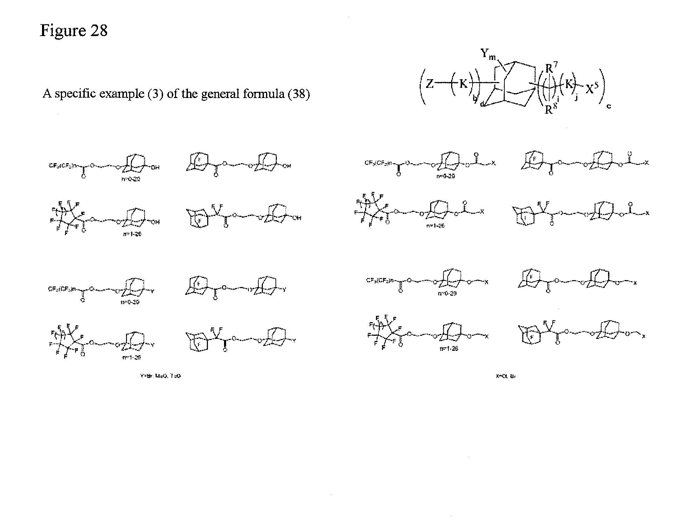

Fig. 28 is a constitutional formula illustrating a specific example (3) of a compound represented by the general formula (38).

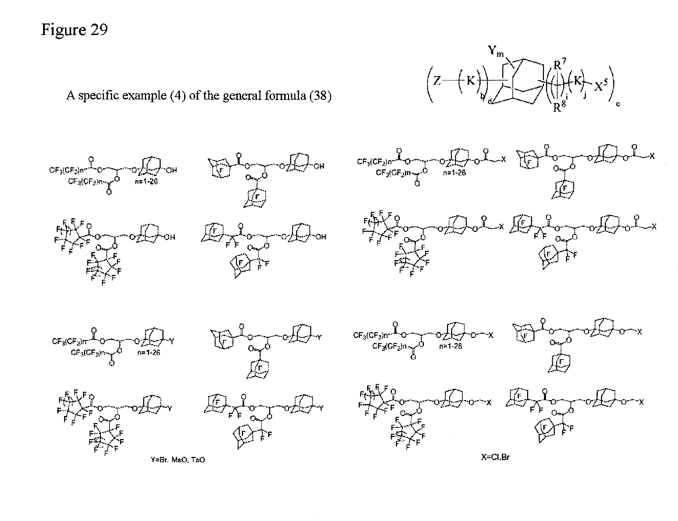

Fig. 29 is a constitutional formula illustrating a specific example (4) of a compound represented by the general formula (38).

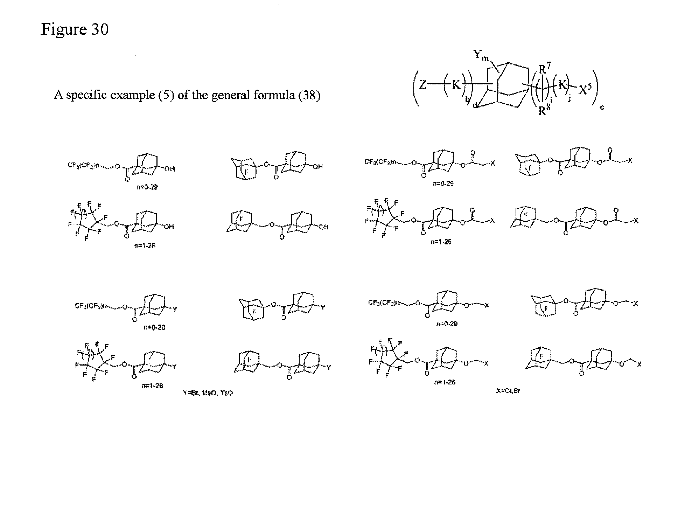

Fig. 30 is a constitutional formula illustrating a specific example (5) of a compound represented by the general formula (38).

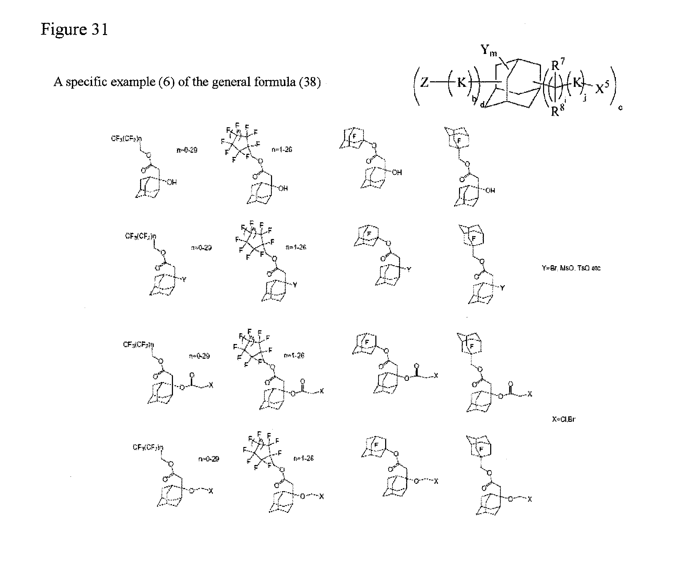

Fig. 31 is a constitutional formula illustrating a specific example (6) of a compound represented by the general formula (38).

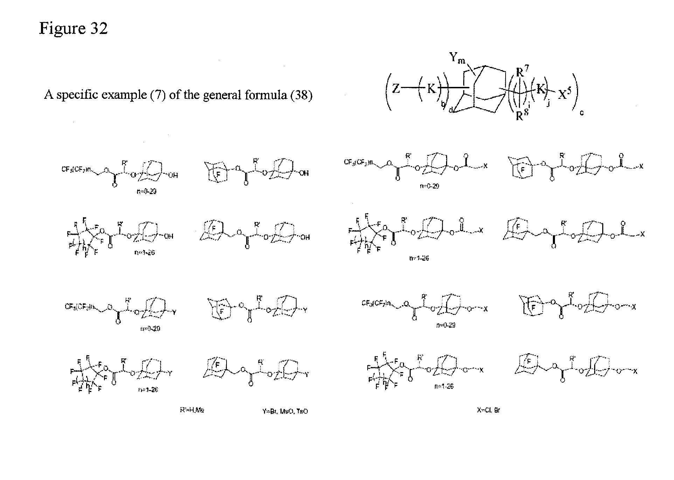

Fig. 32 is a constitutional formula illustrating a specific example (7) of a compound represented by the general formula (38).

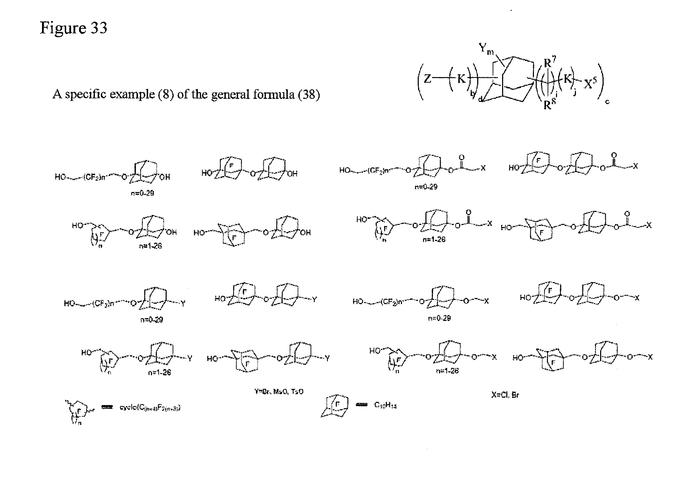

Fig. 33 is a constitutional formula illustrating a specific example (8) of a compound represented by the general formula (38).

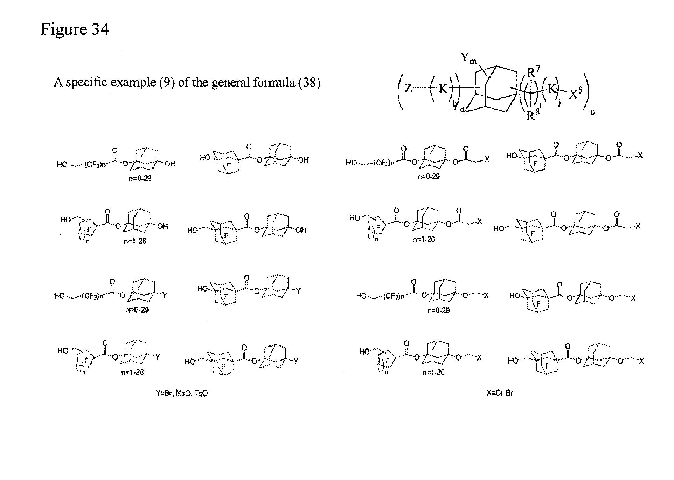

Fig. 34 is a constitutional formula illustrating a specific example (9) of a compound represented by the general formula (38).

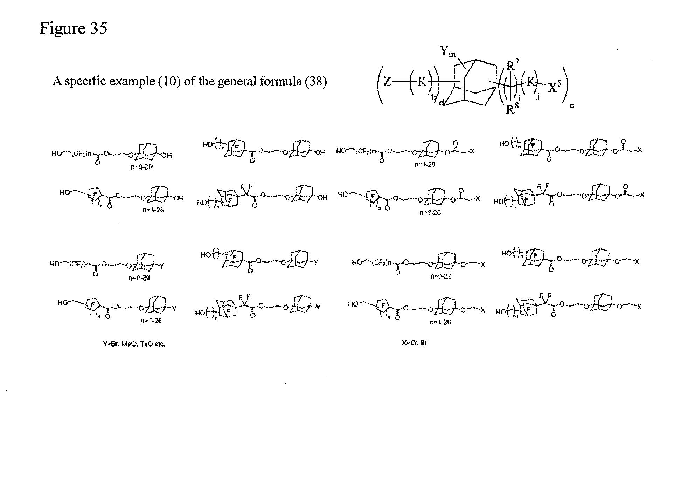

Fig. 35 is a constitutional formula illustrating a specific example (10) of a compound represented by the general formula (38).

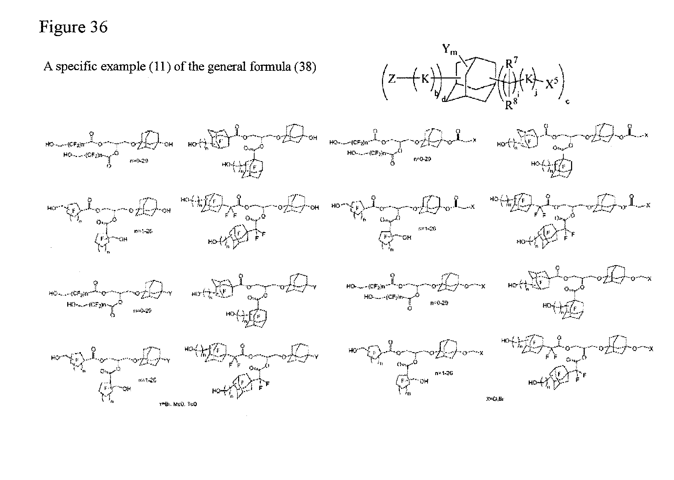

Fig. 36 is a constitutional formula illustrating a specific example (11) of a compound represented by the general formula (38).

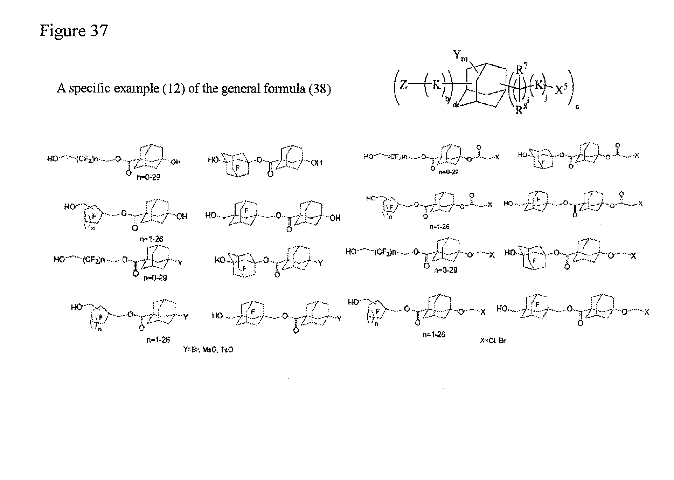

Fig. 37 is a constitutional formula illustrating a specific example (12) of a compound represented by the general formula (38).

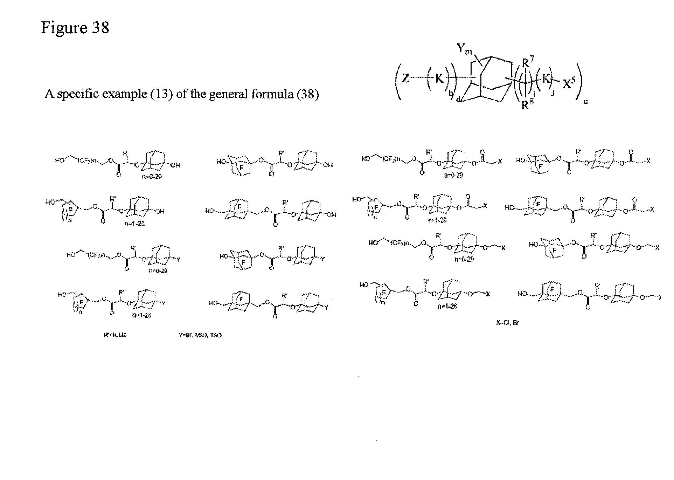

Fig. 38 is a constitutional formula illustrating a specific example (13) of a compound represented by the general formula (38).

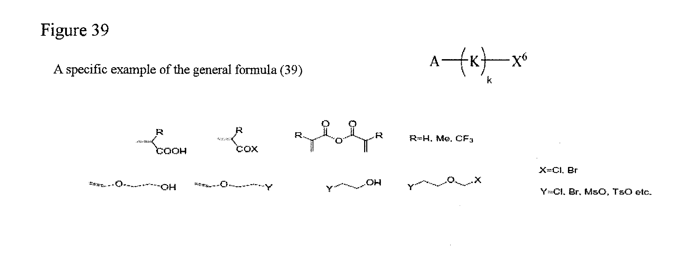

Fig. 39 is a constitutional formula illustrating a specific example of a compound represented by the general formula (39).

### Best Mode for Carrying out the Invention

As described above, the polymerizable compound having the adamantane structure in the present invention is a polymerizable compound having the fluorinated substituent, the adamantane structure and the polymerizable group and at first includes the structure represented by the following general formula (1).

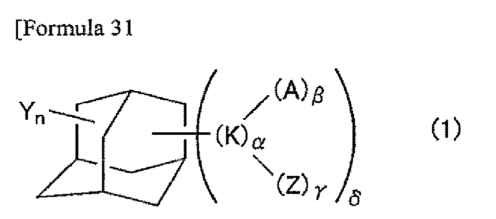

In the formula, A is the group containing the polymerizable group represented by the formula (2) or (3) and a plurality of A may be identical or different. K is a linkage group represented by any one of the formulas (4) to (8) and a plurality of K may be identical or different. Z is the fluorinated substituent described below and a plurality of Z may be identical or different. Y is a substituent on adamantane and represents a hydrogen atom, an alkyl group having 1 to 10 carbon atoms, a halogen atom, hydroxyl, mercapto, and methylcyano groups, and =O or =S formed by joining two Ys. A plurality of Y may also be identical or different. α is an integer of 1 or more and β and γ each are an integer of 0 or more, but one or more of A and Z are included in the general formula (1). δ is an integer of 1 to 16, n is an integer of 0 to 15, and δ + n is equal to 16.

A (polymerizable group):

[Formula 32] CH₂=CR⁰- (2) CH≡C- (3)

R⁰ represents a hydrogen atom, a fluorine atom or a methyl, ethyl or trifluoromethyl group.

K (Linkage group):

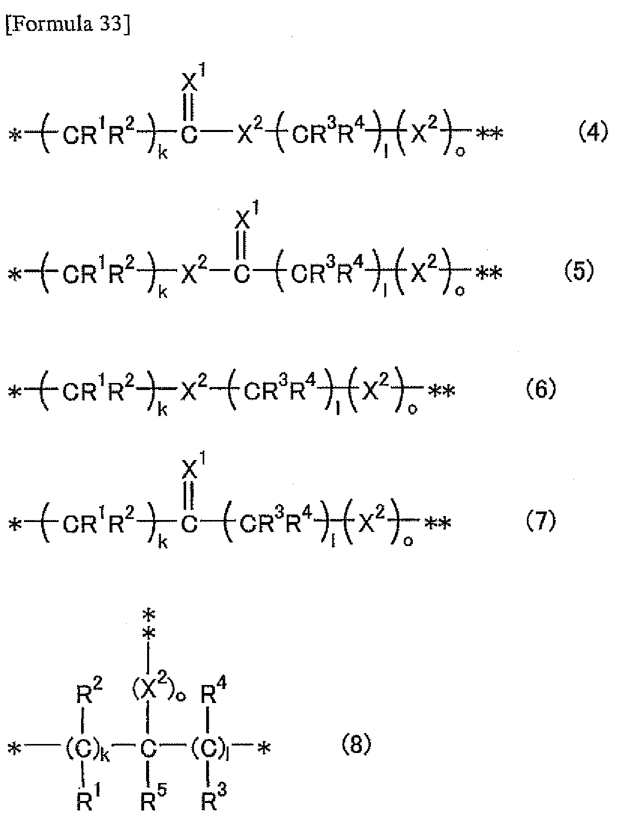

R¹, R², R³, R⁴ and R⁵ each independently represent a hydrogen atom, an alkyl group having 1 to 10 carbon atoms optionally containing a heteroatom or a halogen atom, k and 1 each represent an integer of 0 to 10, X¹ and X² each independently represent an oxygen atom, a sulfur atom or an NR' group. R' is a hydrogen atom or an alkyl group having 1 to 10 carbon atoms optionally containing a heteroatom, single asterisk (*) represents the side of a polymerizable group or the side of an end group, and a double asterisk (**) represents the side of then adamantane ring.

Z (Fluorinated substituent):
Z represents an alkyl group having 1 to 30 carbon atoms or a cycloalkyl group having 5 to 30 carbon atoms, has to include a fluorine atom in part of the structure thereof, and optionally contains in part of the structure thereof at least one kind selected from a heteroatom or hydroxyl, mercapto, ether, thioether, cyano, ketone, thioketone, ketal, thioketal, acetal, thioacetal, lactone, thiolactone, carbonate, thiocarbonate, amine, amide, alkylsulfonyl, ester and thioester groups.)

Such a polymerizable compound having the adamantane structure represented by the general formula (1) can specifically have various structures. For example, the polymerizable compound having the adamantane structure represented by the following general formulas (9) to (13) can be included. In the polymerizable compound having the adamantane structure represented by the following general formula (9), α is 2 or more in the general formula (1) and A (polymerizable group) is linked with K (linkage group) in one side while Z (fluorinated substituent) is linked with K (linkage group) in the other side. a, b, c, d, and f each are an integer of 1 or more, m is an integer of 0 to 14, and c + d + m is equal to 16.

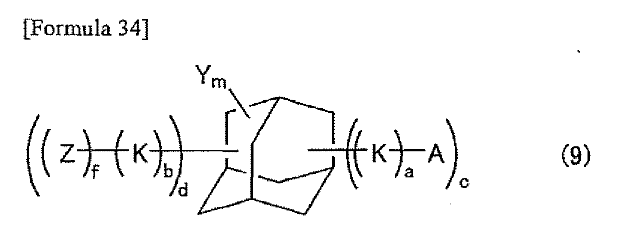

The polymerizable compound having the adamantane structure represented by the following general formula (10) corresponds to the case, where α is also 2 or more in the general formula (1) as similar to the general formula (9) described above and A (polymerizable group) is linked with K (linkage group) in one side while Z (fluorinated substituent) is linked with K (linkage group) in other side, but A is the group containing the polymerizable group represented by the general formula (2). The system containing such a polymerizable group is referred to as an acrylic type. X³ and X⁴ each independently represent an oxygen atom, a sulfur atom or NR' group. R' is a hydrogen atom or an alkyl group having 1 to 10 carbon atoms optionally containing a heteroatom. a, b, c, d, f, and m are similar to those in the general formula (9).

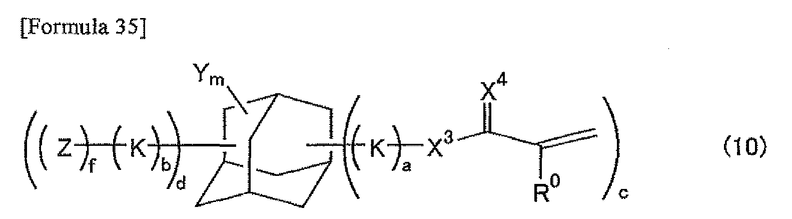

The polymerizable compound having the adamantane structure represented by the following general formula (11) differs in the group containing the polymerizable group represented by the general formula (2) in the general formula (10) described above and such a system is referred to as a vinyl ether type. The polymerizable compound having the adamantane structure represented by the following general formula (12) and the general formula (13) now corresponds to the case, where X³ and X⁴ in the general formula (10) and for X³ in the general formula (11) each have an oxygen atom.

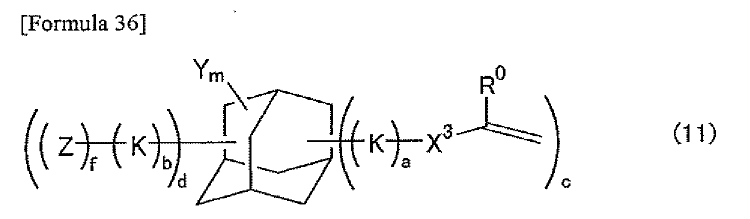

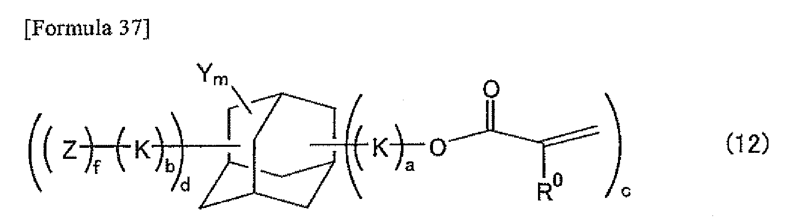

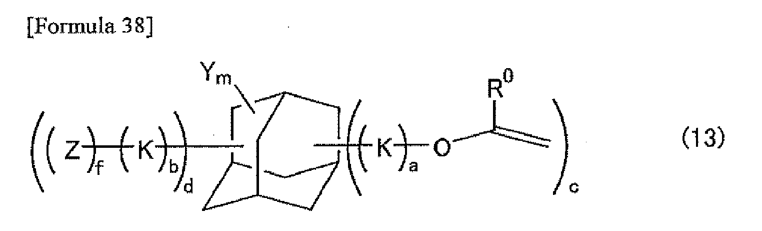

The present invention also provides a polymerizable compound having the adamantane structure represented by the following general formulas (14) to (20) and (27). Such a polymerizable compound having the adamantane structure will be described next.
In the polymerizable compound having the adamantane structure represented by the general formula (14), K (linkage group), Z (fluorinated substituent), K (substituent on adamantane), R⁰, b, c, d, f, and m are similar to those in the general formula (10). R⁷ and R⁸ each independently represent a hydrogen atom, an alkyl group having 1 to 10 carbon atoms optionally containing a heteroatom or a halogen atom. L independently represents a carbon, oxygen, nitrogen or sulfur atom. A plurality of R⁷ and a plurality of R⁸ each may be identical or different. However, when L is an oxygen, nitrogen or sulfur atom, either one or both of R⁷ and R⁸ are absent. e is an integer of 0 to 5.

The polymerizable compound having the adamantane structure represented by the following general formula (14) has in the general formula (12) described above the linkage group (K)ₐ to be e pieces of L (carbon, oxygen, nitrogen or sulfur atom) linked with R⁷ and R⁸ (hydrogen atom, alkyl group having 1 to 10 carbon atoms optionally containing a heteroatom or halogen atom).
The polymerizable compound having the adamantane structure represented by the following general formulas (15) to (19) has the structure with the altered polymerizable group linked to L in the polymerizable compound having the adamantane structure represented by the general formula (14). R⁶ represents a hydrogen atom, an alkyl group having 1 to 10 carbon atoms optionally containing a heteroatom or a halogen atom. L independently represents a carbon, oxygen, nitrogen or sulfur atom.

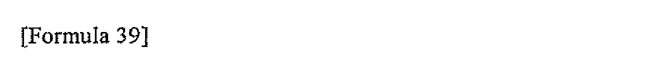

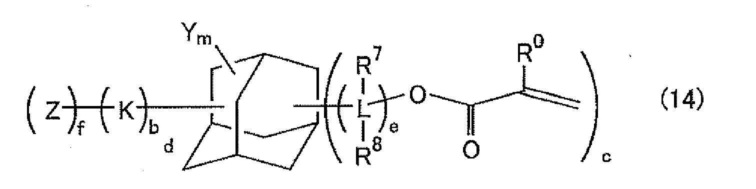

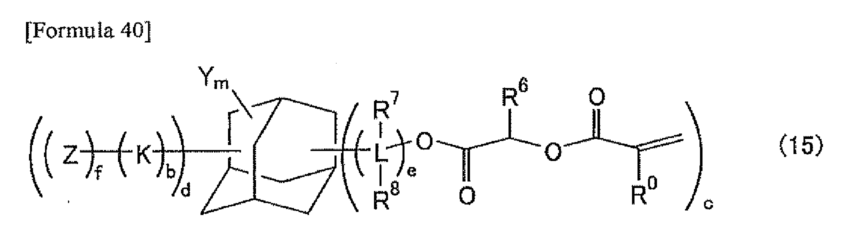

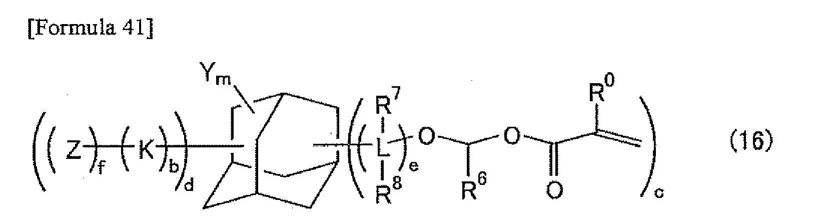

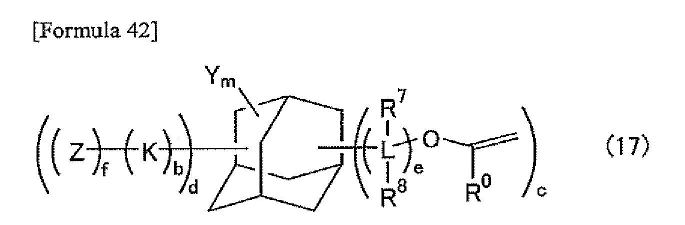

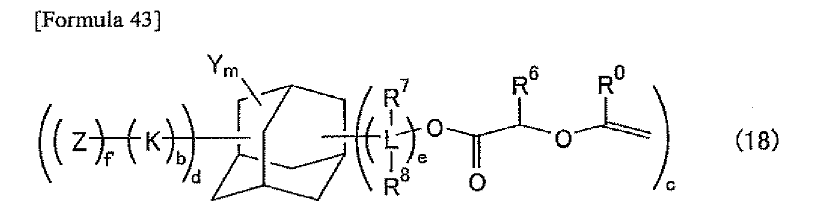

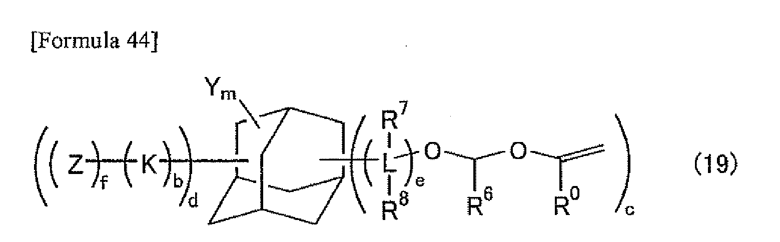

The polymerizable compound having the adamantane structure represented by the following general formula (20) has in the above general formula (14) the linkage group (K)_{b} to be f' pieces of M (carbon, oxygen, nitrogen or sulfur atom) linked with R⁹, R¹⁰, and R¹¹ (hydrogen atom, alkyl group having 1 to 10 carbon atoms optionally containing a heteroatom or halogen atom). A plurality of R⁹ and a plurality of R¹⁰ each may be identical or different. However, when M is an oxygen, nitrogen or sulfur atom, either one or both of R⁹ and R¹⁰ in M are absent. e is an integer of 0 to 5.

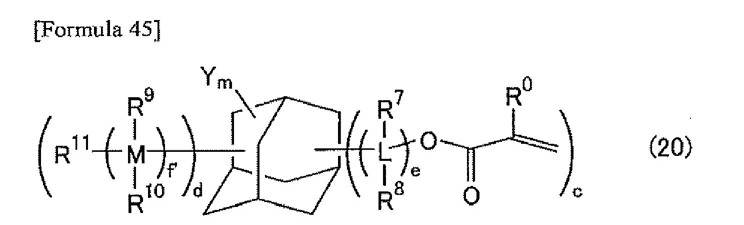

In a linkage of the fluorinated substituent with the polymerizable compound having the adamantane structure represented by this general formula (20) at least one of R⁹ to R¹¹ is any one of the following general formulas (21) to (26). g and h in the following general formula are now an integer of 0 or more.

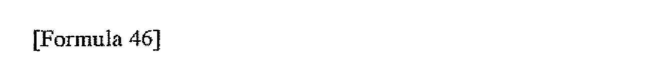

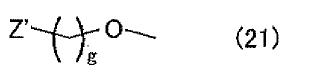

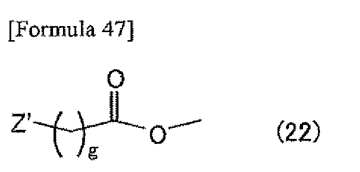

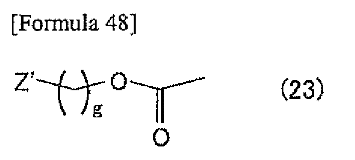

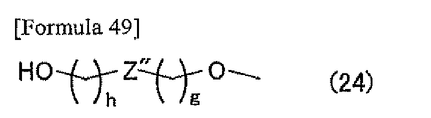

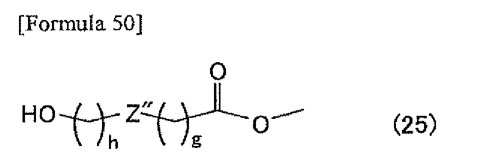

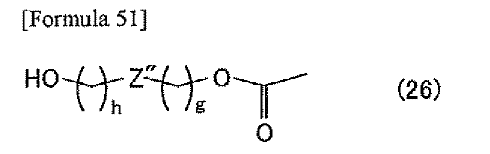

In the polymerizable compound having the adamantane structure represented by the general formula (20), the structure illustrated in Fig. 1 can be included as the polymerizable compound having the adamantane structure having a linkage of the fluorinated substituent represented by the general formula (21).
In the polymerizable compound having the adamantane structure represented by the general formula (20), the structure illustrated in Figs. 2 and 3 can be included as the polymerizable compound having the adamantane structure having a linkage of the fluorinated substituent represented by the general formula (22).
In the polymerizable compound having the adamantane structure represented by the general formula (20), the structure illustrated in Figs. 4 and 5 can be included as the polymerizable compound having the adamantane structure having a linkage of the fluorinated substituent represented by the general formula (23).
In the polymerizable compound having the adamantane structure represented by the general formula (20), the structure illustrated in Fig. 11 can be included as the polymerizable compound having the adamantane structure having a linkage of the fluorinated substituent represented by the general formula (24).
In the polymerizable compound having the adamantane structure represented by the general formula (20), the structure illustrated in Figs. 12 and 13 can be included as the polymerizable compound having the adamantane structure having a linkage of the fluorinated substituent represented by the general formula (25).
In the polymerizable compound having the adamantane structure represented by the general formula (20), the structure illustrated in Figs. 14 and 15 can be included as the polymerizable compound having the adamantane structure having a linkage of the fluorinated substituent represented by the general formula (26).

The polymerizable compound having the adamantane structure represented by the following general formula (27) is obtained by similarly converting the above general formula (17) as the general formula (20).

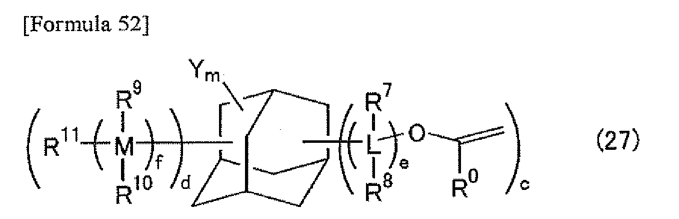

A linkage of the fluorinated substituent with the polymerizable compound having the adamantane structure represented by this general formula (27) is any one of the following general formulas (28) to (33) for at least one of R⁹ to R¹¹ as similar to the general formula (20).

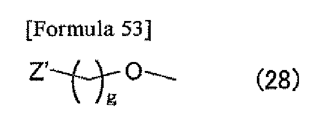

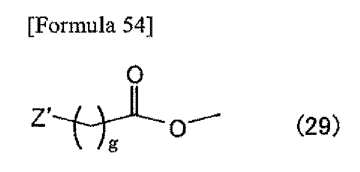

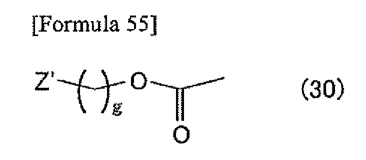

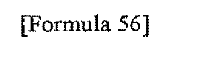

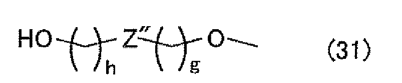

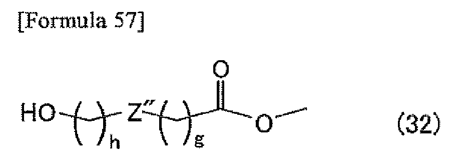

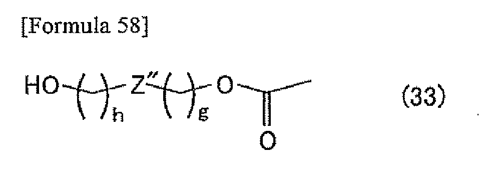

In the polymerizable compound having the adamantane structure represented by the general formula (27), the structure illustrated in Fig. 6 can be included as the polymerizable compound having the adamantane structure with a linkage of the fluorinated substituent represented by the general formula (28).
In the polymerizable compound having the adamantane structure represented by the general formula (27), the structure illustrated in Figs. 7 and 8 can be included as the polymerizable compound having the adamantane structure with a linkage of the fluorinated substituent represented by the general formula (29).
In the polymerizable compound having the adamantane structure represented by the general formula (27), the structure illustrated in Figs. 9 and 10 can be included as the polymerizable compound having the adamantane structure with a linkage of the fluorinated substituent represented by the general formula (30).
In the polymerizable compound having the adamantane structure represented by the general formula (27), the structure illustrated in Fig. 16 can be included as the polymerizable compound having the adamantane structure with a linkage of the fluorinated substituent represented by the general formula (31).
In the polymerizable compound having the adamantane structure represented by the general formula (27), the structure illustrated in Figs. 17 and 18 can be included as the polymerizable compound having the adamantane structure with a linkage of the fluorinated substituent represented by the general formula (32).
In the polymerizable compound having the adamantane structure represented by the general formula (27), the structure illustrated in Figs. 19 and 20 can be included as the polymerizable compound having the adamantane structure with a linkage of the fluorinated substituent represented by the general formula (33).

A production method of the polymerizable compound having the adamantane structure in the present invention will be described next. The first production method relates to the production method of the polymerizable compound having the adamantane structure represented by the general formula (37), in which a compound of the general formula (34), in which at least one of R⁹, R¹⁰, and R¹² is represented by the general formula (35) is reacted with a compound represented by the general formula (36).

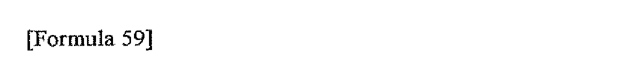

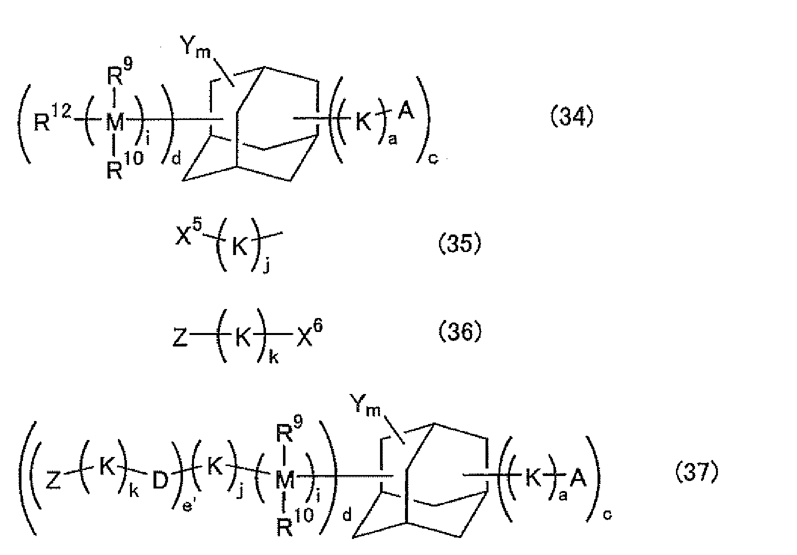

A (polymerizable group), K (linkage group), Z (fluorinated substituent), Y (substituent on adamantane), and m are similar to those in the general formula (9).
R⁹, R¹⁰, and R¹² each independently represent a hydrogen atom, an alkyl group having 1 to 10 carbon atoms optionally containing a heteroatom or a halogen atom. A plurality of R⁹, a plurality of R¹⁰ and a plurality of R¹² each may be identical or different.
M independently represents a carbon, oxygen, nitrogen or sulfur atom. However, when M is an oxygen, nitrogen or sulfur atom, either one or both of R⁹ and R¹⁰ are absent.
X⁵ and X⁶ are a reactive group, and when X⁵ is selected from a hydrogen atom, a halogen atom, an alkylsulfonyloxy, perfluoroalkylsulfonyloxy or alkyl-substituted phenylsulfonyloxy group, X⁶ represents a hydrogen atom or a group selected from hydroxyl, mercapto, or amino group or salt thereof whereas when X⁵ is selected from a hydrogen atom, a hydroxyl, mercapto or amino group or salt thereof, X⁶ represents a hydrogen atom, a halogen atom or a group selected from an alkylsulfonyloxy, perfluoroalkylsulfonyloxy or alkyl-substituted phenylsulfonyloxy group. D represents a linkage group formed by the reaction of X⁵ with X⁶. a, c, d and e' each are an integer of 1 or more, i is an integer of 0 to 5, j and k each are an integer of 0 or more and c + d + m is equal to 16.

A specific example of the general formula (34), in which at least one of R⁹, R¹⁰, and R¹² is represented by the general formula (35) includes the structure described in Figs. 21 to 24. Among them, Figs. 21 to 22 correspond to the acrylate, Fig. 23 corresponds to the vinyl ether, and Fig. 24 corresponds to the vinyl ether precursor. A specific example of a compound represented by the general formula (36) includes the structure described in Fig. 25.
Various reactions are possible between a compound of the general formula (34) in which at least one of R⁹, R¹⁰, and R¹² is represented by the general formula (35) and a compound of the general formula (36). An example of (A) etherification and (B) esterification will be described herein.

(A) Etherification
In the above reaction equation, when X⁵ in the general formula (35) is selected from a halogen atom, an alkylsulfonyloxy, perfluoroalkylsulfonyloxy or alkyl-substituted phenylsulfonyl group and the like, X⁶ in the general formula (36) is selected from a compound having at least one hydroxyl, mercapto or amino group or salt thereof. When X⁵ in the general formula (35) is selected from a hydroxy, mercapto or amino group or a salt thereof, X⁶ in the general formula (36) is also selected from a compound having at least one halogen atom, an alkylsulfonyloxy, perfluoroalkylsulfonyloxy or alkyl-substituted phenylsulfonyloxy group and the like.
The reaction temperature is in the range of -200 to 200°C, preferably -20 to 150°C. When the temperature is too low, the reaction rate is decreased to prolong the reaction time. When the temperature is too high, formation of polymer byproducts is increased. The reaction pressured is in the range of 0.01 to 10 MPa as an absolute pressure, preferably a normal pressure to 10 MPa. When the pressure is too high, special equipment is necessary and uneconomic. The reaction time is in the range of 1 to 48 hours.

In this etherification reaction, a base can be added as needed. The kind of base includes sodium amide, triethylamine, tributylamine, trioctylamine, pyridine, N,N-dimethylaniline, 1,5-diazabicylo[4.3.0]-5-nonene (DBN), 1,8-diazabicylo[5.4,0]-7-undecene (DBU), sodium hydroxide, potassium hydroxide, sodium hydride, potassium carbonate, silver oxide, sodium methoxide, potassium t-butoxide, sodium phosphate, sodium monohydrogenphosphate, sodium dihydrogenphosphate and the like.
A solvent may not be present, but a solvent with the solubility of a compound represented by the general formula (34), in which at least one of R⁹, R¹⁰, and R¹² is represented by the general formula (35) and a compound represented by the general formula (36) in the general formula (34) to be 0.5% by mass or more, preferably 5% by mass or more is preferably used. The amount of the solvent is adjusted to such an amount that the concentration of a compound represented by the general formula (34), in which at least one of R⁹, R¹⁰, and R¹² is represented by the general formula (35) and a compound represented by the general formula (36) is generally 0.5% by mass or more, and preferably 5% by mass or more in the reaction mixture. In this case, the compound represented by the general formula (34), in which at least one of R⁹, R¹⁰, and R¹² is represented by the general formula (35) and a compound represented by the general formula (36) may be in a state of suspension, but preferably dissolved. Residual water in the solvent is also preferably removed prior to use. The solvent specifically includes a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, tetrahydrofuran (THF), dioxane and the like, a halogenated solvent such as dichloromethane, carbon tetrachloride and the like, dimethylsulfoxide, N,N-dimethylformamide, N-methylpyrrolidone, γ-butyrolactone and the like.
Distillation, crystallization, column separation and the like can be used as a purification method, which can be selected depending on the properties of the product and kinds of impurities.

(B) Esterification
In a case of esterification, reaction can be performed under a condition similar to the general esterification. Specifically, (i) reaction of a carboxylic acid with an alcohol, (ii) reaction of a carboxylic acid halide with an alcohol or salt thereof, (iii) reaction of a carboxylic anhydride with an alcohol or salt thereof and the like can be included. When a compound represented by the general formula (34), in which at least one of R⁹, R¹⁰, and R¹² is represented by the general formula (35) is selected from a carboxylic acid or reactive derivative thereof, for example, a carboxylic acid halide or carboxylic anhydride, a compound represented by the general formula (36) is selected from an alcohol or salt thereof, whereas when a compound represented by the general formula (36) is selected from a carboxylic acid or reactive derivative thereof, for example, a carboxylic acid halide or carboxylic anhydride, a compound represented by the general formula (34), in which at least one of R⁹, R¹⁰, and R¹² is represented by the general formula (35) is selected from an alcohol or salt thereof.

(i) Reaction of a carboxylic acid with an alcohol (RCOOH + R'OH)
A general method such as azeotropic dehydration, a method using a dehydrating agent such as dicyclohexylcarbodiimide (DCC) and the like, a method using trifluoroacetic anhydride and the like can be used.
The optimum reaction temperature differs depending on which method among the above methods is used, but the reaction temperature is generally in the range of -200 to 200°C, and preferably -20 to 150°C. When the temperature is too low, the reaction rate is decreased to prolong the reaction time. When the temperature is too high, formation of polymer byproducts is increased.
The reaction pressure is generally in the range of 0.01 to 10 MPa as an absolute pressure, preferably a normal pressure to 10 MPa. When the pressure is too high, a special equipment is necessary and uneconomic. The reaction time is in the range of 1 to 48 hours.

An additive such as a catalyst and the like can be used as needed. Such an additive differs with which method among the above reaction methods is used. In the case of azeotropic dehydration, an acid catalyst such as hydrochloric acid, sulfuric acid, methanesulfonic acid, para-toluenesulfonic acid, methanesulfonic acid and the like can be used. The amount added is in the range of 0.1 to 50 mol%, and preferably 1 to 10 mol%. In the case of a dehydrating agent method, N,N-dimethylpyridine, pyridine, triethylamine, 1,5-diazabicylo[4.3.0]-5-nonene (DBN), 1,8-diazabicylo[5.4.0]-7-undecene (DBU) and the like can used as a catalyst. The amount added is in the range of 0.1 to 100 mol%, and preferably 1 to 20 mol%. In the case of the method using trifluoroacetic anhydride, sodium amide, triethylamine, tributylamine, trioctylamine, pyridine, N,N-dimethylaniline, 1,5-diazabicylo[4.3.0]-5-nonene (DBN), 1,8-diazabicylo[5.4.0]-7-undecene (DBU), sodium hydroxide, potassium hydroxide, sodium hydride, potassium carbonate, silver oxide, sodium methoxide, potassium t-butoxide, sodium phosphate, sodium monohydrogenphosphate, sodium dihydrogenphosphate and the like can be used as a trapping agent of trifluoroacetic acid formed as a byproduct. The amount added is in the range of 50 to 300 mol%, and preferably 100 to 200 mol%.

A solvent may not be present, but a suitable solvent can be selected depending on which method among the above the reaction methods is used. A solvent with the solubility of a compound represented by the general formula (34), in which at least one of R⁹, R¹⁰, and R¹² is represented by the general formula (35) and a compound represented by the general formula (36) to be generally 0.5% by mass or more, and preferably 5% by mass or more. The amount of the solvent is adjusted to such an amount that the concentration of a compound represented by the general formula (34), in which at least one of R⁹, R¹⁰, and R¹² is represented by the general formula (35) and a compound represented by the general formula (36) is generally 0.5% by mass or more, and preferably 5% by mass or more in the reaction mixture. In this case, the compound represented by the general formula (34), in which at least one af R⁹, R¹⁰, and R¹² is represented by the general formula (35) and a compound represented by the general formula (36) may be in a state of suspension, but preferably dissolved. In the case of azeotropic dehydration, a solvent substantially immiscible with water can be selected. The solvent specifically includes a hydrocarbon solvent such as hexane, heptane, cyclohexane, methylcyclohexane, ethylcyclohexane and the like and an aromatic hydrocarbon solvent such as benzene, toluene, xylene and the like. In the case of the dehydrating agent method or the method using trifluoroacetic anhydride, residual water in the solvent is preferably removed prior to use. Specifically a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, THF, dioxane and the like, a halogenated solvent such as dichloromethane, carbon tetrachloride and the like, dimethylsulfoxide, N,N-dimethylformamide, N-methylpyrrolidone, γ-butyrolactone and the like can be included.
Distillation, crystallization, column separation and the like can be used as a purification method, which can be selected depending on the properties of a product and kinds of impurities.

(ii) Reaction of a carboxylic acid halide or carboxylic anhydride with an alcohol or salt thereof (RCOX + R'OH (M) or (RCO)₂O + R'OH (M))
The reaction temperature is generally in the range of -200 to 200°C, and preferably -20 to 150°C. When the reaction temperature is too low, the reaction rate is decreased to prolong the reaction time. When the temperature is too high, formation of polymer byproducts is increased. The reaction pressure is in the range of 0.01 to 10MPa as an absolute pressure, and preferably normal pressure to 10MPa. When the pressure is too high, a special equipment is necessary and uneconomic. The reaction time is in the range of 1 to 48 hours.
In this reaction, a base can be added as needed. The kind of a base includes sodium amide, triethylamine, tributylamine, trioctylamine, pyridine, N,N-dimethylaniline, 1,5-diazabicylo[4.3.0]-5-nonene (DBN), 1,8-diazabicylo[5.4.0]-7-undecene (DBU), sodium hydroxide, potassium hydroxide, sodium hydride, potassium carbonate, silver oxide, sodium methoxide, potassium t-butoxide, sodium phosphate, sodium monohydrogenphosphate, sodium dihydrogenphosphate and the like.

A solvent may not be present, but a suitable solvent can be selected depending on which method among the above reaction method is used. A solvent with the solubility of a compound represented by the formulas (34) and (36) to be 0.5% or more, and preferably 5% or more is used. The amount of the solvent is adjusted to such an amount that the concentration of a compound represented by the formulas (34) and (36) is 0.5% or more, and preferably 5% or more in the reaction mixture. In this case, the compound represented by the formulas (34) or (36) may be in a state of suspension, but preferably dissolved. The solvent specifically includes a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, THF, dioxane and the like, a halogenated solvent such as dichloromethane, carbon tetrachloride and the like, dimethylsulfoxide, N,N-dimethylformamide, N-methylpyrrolidone, γ-butyrolactone and the like can be included.
Distillation, crystallization, column separation and the like can be used as a purification method, which can be selected depending on the properties of a product and kinds of impurities.

A second production method is the production method of a polymerizable compound having the adamantane structure represented by the general formula (40), in which a compound represented by the general formula (38) is reacted with a compound represented by the general formula (39).

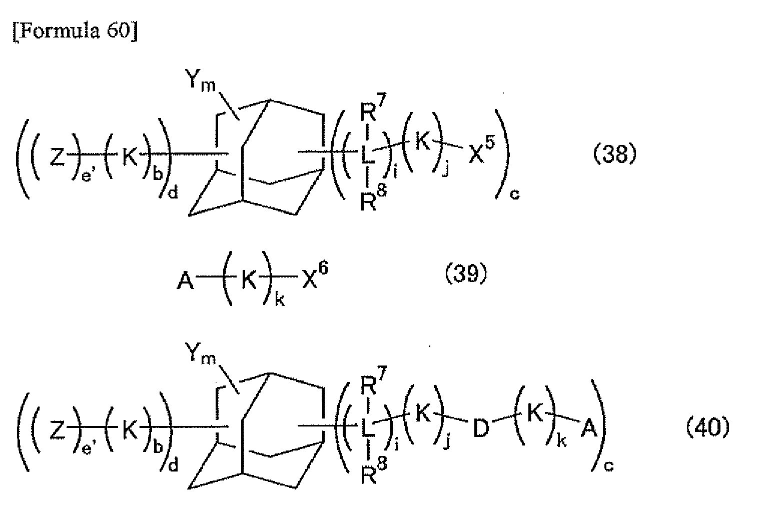

A specific example of a compound represented by the general formula (38) includes the structure described in Figs. 26 to 38. A specific example of a compound represented by the general formula (39) includes the structure described in Fig. 39.
Various reactions are allowed between a compound of the general formula (38) and that of the general formula (39). Derivatization to (A) acrylates (acrylate, methacrylate and α-trifluoromethyl acrylate) and to (B) vinyl ethers and (C) conversion to vinyl ethers will be described herein as an example.

Derivatization to acrylates
Derivatization to these acrylates can be performed under a condition similar to the general esterification. Specifically, (i) reaction of acrylic acids with an alcohol, (ii) reaction of acrylic acids with α-haloacetic acid esters represented by the following general formula (41), (iii) reaction of acrylic acids with halomethyl ethers represented by the following general formula (42), (iv) reaction of an acrylic acid halide with an alcohol or salt thereof, (v) reaction of an acrylic acid anhydride with an alcohol or salt thereof and the like can be included.

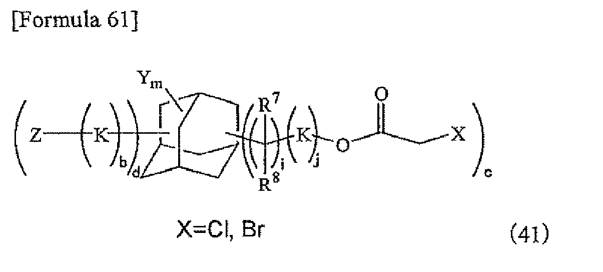

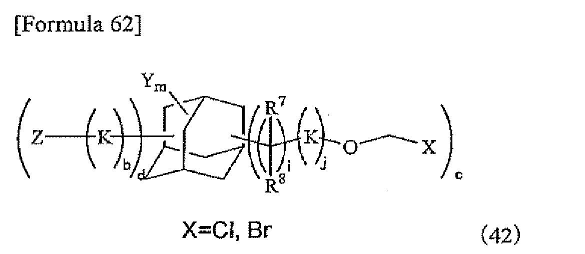

(i) In case of the reaction of acrylic acids with an alcohol
Various conditions are similar to the case of (i) the reaction of a carboxylic acid with an alcohol (RCOOH + R'OH) in the esterification in the preceding paragraph (B).

(ii) In case of the reaction of acrylic acids with α-haloacetic acid esters
An adamantyl haloalkylcarboxylate is preferably reacted with a (meth)acrylate in the presence of a reaction accelerator.
The reaction temperature is in the range of -200 to 200°C, and preferably room temperature to 50°C. When the temperature is too low, the reaction rate is decreased to prolong the reaction time. When the temperature is too high, formation of polymer byproducts is increased. The reaction pressure is in the range of 0.01 to 10 MPa as an absolute pressure, and preferably normal pressure to 1 MPa. When the pressure is too high, a special equipment is necessary and uneconomic. The reaction time is in the range of 1 to 24 hours, and preferably 30 minutes to 6 hours.
A reaction accelerator may not be used, but potassium iodide, trimethylamine, triethylamine, tributylamine, trioctylamine, pyridine, lithium carbonate, potassium carbonate, sodium carbonate and the like are used as needed.

A solvent may not be used, but a solvent with the solubility of a compound represented by the general formula (38) and the general formula (39) to be generally 0.5% by mass or more, preferably 5% by mass or more is preferably used as needed. The amount of the solvent is adjusted to such an amount that the concentration of the compound represented by the general formula (38) and the general formula (39) is generally 0.5% by mass or more, and preferably 5% by mass or more in the reaction mixture. In this case, the compound represented by the general formula (38) and the general formula (39) may be in a state of suspension, but preferably dissolved. Specifically, a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, THF, dioxane and the like, a halogenated solvent such as dichloromethane, carbon tetrachloride and the like, dimethylsulfoxide, N,N-dimethylformamide, N-methylpyrrolidone, γ-butyrolactone and the like can be included.
Distillation, crystallization, column separation and the like are allowed as a purification method, which can be selected depending on the properties of a product and kinds of impurities.

(iii) Reaction of acrylic acids with halomethyl ethers
Halomethyl ethers are reacted with acrylates in the presence of a reaction accelerator, followed by synthesis of adamantyloxymethyl (meth)acrylates.
The reaction temperature is generally in the range of -200 to 200°C, and preferably room temperature to 50°C. When the temperature is too low, the reaction rate is decreased to prolong the reaction time. When the temperature is too high, formation of polymer byproducts is increased. The reaction pressure is in the range of 0.01 to 10MPa as an absolute pressure, and preferably normal pressure to 1MPa. When the pressure is too high, a special equipment is necessary and uneconomic. The reaction time is in the range of 1 minute to 24 hours, and preferably 30 minutes to 6 hours.

The reaction accelerator may not be used, but trimethylamine, triethylamine, tributylamine, trioctylamine, pyridine, lithium carbonate, potassium carbonate, sodium carbonate and the like are used as needed.
A solvent may not be used, but a solvent with the solubility of a compound represented by the general formula (38) and the general formula (39) to be generally 0.5% by mass or more, and preferably 5% by mass or more is used as needed. The amount of the solvent is adjusted to such an amount that the concentration of a compound represented by the general formula (38) and the general formula (39) is generally 0.5% by mass or more, and preferably 5% by mass or more in the reaction mixture. In this case, the compound represented by the general formula (38) and the general formula (39) may be in a state of suspension, but preferably dissolved. Specifically, a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, THF, dioxane and the like, a halogenated solvent such as dichloromethane, carbon tetrachloride and the like, dimethylsulfoxide, N,N-dimethylformamide, N-methylpyrrolidone, γ-butyrolactone and the like can be included.
Distillation, crystallization, column separation and the like are allowed as a purification method, which can be selected depending on the properties of a product and kinds of impurities.

(iv) Reaction of acrylic acid halides with an alcohol or salt thereof and (v) reaction of acrylic anhydrides with an alcohol or salt thereof
The reaction conditions are similar to the case of (i) reaction of a carboxylic acid with an alcohol (RCOOH + R'OH) in the case of esterification (B) in the preceding paragraph.

(B) Derivatization to vinyl ethers
In the general formula (38) and the general formula (39) described above, when X⁵ is selected from a halogen atom, an alkylsulfonyloxy, perfluoroalkylsulfonyloxy, alkyl-substituted phenyl sulfonyloxy group and the like, a compound represented by the general formula (39) is selected from a compound having at least one hydroxyl, mercapto or amino group or salt thereof When X⁵ is selected from a hydroxyl, mercapto, or amino group or salt thereof, a compound represented by the general formula (39) is also selected from a compound having at least one halogen atom, alkylsulfonyloxy, perfluoroalkylsulfonyloxy, alkyl-substituted phenylsulfonyloxy group and the like. The reaction conditions such as the reaction temperature, pressure and the like are similar to the case of etherification (A) in the preceding paragraph.

A special example in the above reaction includes (i) the reaction of a compound having a vinyl ether group or a group convertible to the vinyl ether group represented by the general formula (38) with α-haloacetic acid esters represented by the general formula (41), (ii) the reaction of a compound having the vinyl ether group or a group convertible to the vinyl ether group represented by the general formula (38) with halomethyl ethers represented by the general formula (42), and the like.
Reaction conditions such as the temperature, pressure, and the like in (i) the reaction of a compound having the vinyl ether group or a group convertible to the vinyl ether group represented by the general formula (38) with the α-haloacetic acid esters represented by the general formula (41) are similar to the case (ii) in the reaction of the acrylic acids with the α-haloacetic acid esters described above.
The reaction conditions such as the temperature, pressure, and the like in (ii) the reaction of a compound having the vinyl ether group or a group convertible to the vinyl ether group represented by the general formula (38) with the halomethyl ethers represented by the general formula (42) are similar to the case (iii) in the reaction of the acrylic acids with the halomethyl ethers described above.

(C) Conversion to vinyl ether group
Conversion to the vinyl ether group in the presence of a base is allowed according to the following reaction equation. 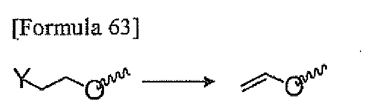 (In the formula, Y represents a halogen atom, an alkylsulfonyloxy, perfluoroalkylsulfonyloxy or alkyl-substituted phenylsulfonyloxy group and the like.)

The reaction temperature is in the range of -200 to 200°C, and preferably 0 to 100°C. When the temperature is too low, the reaction rate is decreased to prolong the reaction time. When the temperature is too high, the side reaction is potentially increased. The reaction pressure is in the range of 0.01 to 10 MPa as an absolute pressure, and preferably a normal pressure to 1 MPa. When the pressure is too low, the reaction time is prolonged, while when the pressure is too high, a special equipment is necessary and uneconomic. The reaction time is in the range of 1 minute to 24 hours, and preferably 1 to 6 hours.
A base used includes sodium amide, triethylamine, pyridine, N,N-dimethylaniline, 1,5-diazabicylo[4.3.0]-5-nonene (DBN), 1,8-diazabicylo[5.4.0]-7-undecene (DBU), sodium hydroxide, potassium hydroxide, sodium hydride, potassium carbonate, silver oxide, sodium methoxide, potassium t-butoxide, and the like.
A solvent may not be used, but a solvent with the solubility of a compound represented by the general formula (38) and the general formula (39) to be generally 0.5% by mass or more, and preferably 5% by mass or more is used as needed. The amount of the solvent is adjusted to such an amount that the concentration of a compound represented by the general formula (38) and the general formula (39) is generally 0.5% by mass or more, and preferably 5% by mass or more in the reaction mixture. In this case, the compound represented by the general formula (38) and the general formula (39) may be in a state of suspension, but preferably dissolved. Specifically, a hydrocarbon solvent such as hexane, heptane, cyclohexane, methylcyclohexane, ethylcyclohexane and the like, an aromatic hydrocarbon solvent such as benzene, toluene, xylene and the like, an ether solvent such as diethyl ether, THF, dioxane and the like, a halogenated solvent such as dichloromethane, carbon tetrachloride and the like, dimethylsulfoxide, N,N-dimethylformamide, N-methylpyrrolidone, γ-butyrolactone and the like can be included.
Distillation, crystallization, column separation and the like are allowed as a purification method, which can be selected depending on the properties of a product and kinds of impurities.

The present invention further provides a polymer constituted of the various polymerizable compounds having the adamantane structure described above in the present invention, a photoresist composition containing the polymer, a thermocurable resin composition constituted of the polymerizable compound having the adamantane structure and a photocurable resin composition constituted of the polymerizable compound having the adamantane structure.

In case of the photoresist composition, the constituent thereof is a polymer obtained by copolymerizing the polymerizable compound having the adamantane structure with an acid-decomposing monomer such as 2-methyl-2-adamantyl (meth)acrylate, a monomer containing a polar group such as 3-hydroxy-1-adamantyl (meth)acrylate and a lactone-containing monomer such as γ-butyrolactone-2-(meth)acrylate, a photoacid generator, a quencher, a solvent and other additives.

In case of the thermocurable resin composition, the constituent thereof is the polymerizable compound having the adamantane structure in the present invention, a monomer copolymerizable with this compound, a heat polymerization initiator and other additives. A solvent may be added as needed.

In case of the photocurable resin composition, the constituent thereof is the polymerizable compound having the adamantane structure in the present invention, a monomer copolymerizable with this compound, a photopolymerization initiator and other additives. A photosensitizer and a solvent may be added as needed.

### Examples

The present invention will be next described in more detail according to Example, but not limited in any way by these Examples.

### Example 1

Synthesis of 3-[(undecafluorocyclohexyl)methoxy]-1-adamantyl methacrylate represented by the following formula

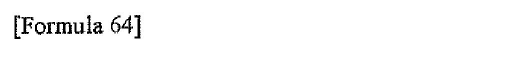

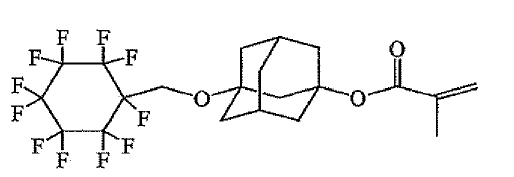

Into a 100 mL flask equipped with an agitator, a thermometer and an air introduction tube were charged 3-methanesulfonyloxy-1-adamantyl methacrylate (molecular weight: 314.40, 20 mmol, 6.29 g, manufactured by Idemitsu Kosan Co., Ltd.), (undecafluorocyclohexyl)methanol (molecular weight: 312.08, 40 mmol, 12.48g), sodium dihydrogenphosphate (molecular weight: 141.96, 80 mmol, 11.36g), γ-butyrolactone (45mL), and methoquinone (6.3 mg). Agitation of the mixture was initiated while introducing air into it as well as the flask was immersed in an oil bath to raise the temperature to 120°C. After 6 hours, the reaction solution was analyzed by gas chromatography confirming the disappearance of 3-methanesulfonyloxy-1-adamantyl methacrylate. The reaction solution was cooled, followed by the addition of 100 mL of deionized water to yield a homogeneous solution, which was then transferred to a separation funnel and extracted three times with 60 mL of hexane. An organic layer was combined, washed with deionized water and saturated brine and the organic layer was then separated and dried after addition of magnesium sulfate. After filtration of magnesium sulfate, the solvent was evaporated to yield a crude product. This product was chromatographed on a silica gel column to yield targeted 3-[(undecafluorocyclohexyl)methoxy]-1-adamantyl methacrylate (molecular weight: 530.37, yield 6.50 g, factional yield 61.3%).

### <Spectral data>

The results of the measurement of a nuclear magnetic resonance spectrum of 3-[(undecafluorocyclohexyl)methoxy]-1-adamantyl methacrylate obtained are as follows. (JNM-ECA500 manufactured by JEOL, Ltd. was used. Solvent: deuterochloroform.) ¹H-NMR (500MHz): 1.56(dd,2H), 1.76(dd,4H), 1.89(s,3H), 2.10(dd,4H), 2.20(s,2H), 2.39(s,2H), 4.16(d,2H), 5.50(t,1H), 6.01(s,1H)
¹³C-NMR (125MHz): 18.30, 30.90, 34.88, 39.81, 40.01, 44.60, 76.25, 81.00, 124.85, 137.62, 166.40
¹⁹F-NMR (470MHz): -114.16(1F), -66.47(d,lF), -64.28(d,2F), -56.23(d,2F), -48.84(d,1F), -47.30(d,2F), -43.60(d,2F)
GC-MS (EI): (GCMS-QP2010 manufactured by Shimadzu Corp. was used.) s30(M+-, 6.6%), 444(44.20%), 402(15.2%), 389(49.5%), 376(18.5%), 219(7.1%), 133(9.2%), 121(4.2%), 105(6.1%), 92(100%), 69(53.7%), 55(6.9%)

### Example 2

### Synthesis of 3-{[1,2,2,3,3,4,5,5,6,6-decafluoro-4-(hydroxymethyl)cyclohexyl]methoxy}-1-adamantyl methacrylate represented by the following formula

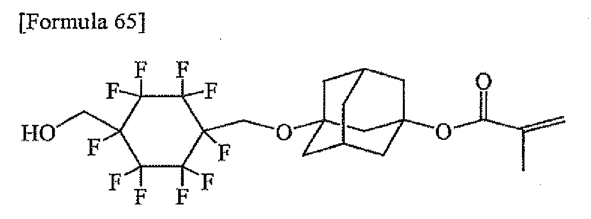

The reaction was carried out by a method similar to Example 1 except perfluorocyclohexane-1,4-dimethanol (molecular weight: 314.12, 40 mmol, 12.97 g) was used instead of (undecafluorocyclohexyl)methanol. GC-MS analysis confirmed the formation of the targeted product (molecular weight: 542.41).

### <Spectral data>

GC-MS (EI): (GCMS-QP2010 manufactured by Shimadzu Corp. was used.)
542(M+, 2.2%), 456(32.3%), 414(11.3%), 401(36.1%), 388(13.5%), 219(5.5%), 133(10.4%), 92(100%), 79(11.9%), 69(64.7%), 55(7.5%), 41(45.3%)

### Example 3

### Synthesis of 3-{[1,2,2,3,3,4,4,5,5,6-decafluoro-6-(hydroxymethyl)cyclohexyl]methoxy}-1-adamantyl methacrylate represented by the following formula

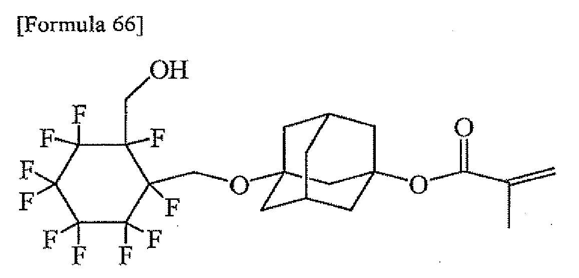

The reaction was carried out by a method similar to Example 1 except perfluorocyclohexane-1,2-dimethanol (molecular weight: 314.12, 40 mmol, 12.97 g) was used instead of (undecafluorocyclohexyl)methanol. GC-MS analysis confirmed the formation of the targeted product (molecular weight: 542.41).

### <Spectral data>

GC-MS (EI): (GCMS-QP2010 manufactured by Shimadzu Corp. was used.)
542(M+, 0.5%), 456(31.9%), 401(12.5%), 382(10.7%), 219(18.2%), 133(14.9%), 108(12.4%), 92(95.1%), 69(100%), 55(8.4%), 41(51.3%)

### Example 4

### Synthesis of 3-[2,2,3,3,4,4,5,5,6,6,6-undecafluorohexyl)oxy]-1-adamantyl methacrylate represented by the following formula

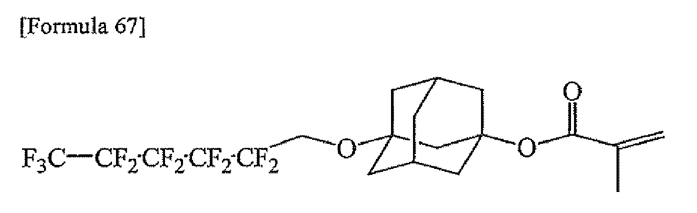

The reaction was carried out by a method similar to Example 1 except 1H, 1H-perfluorohexanol (molecular weight: 300.07, 40 mmol, 12.00 g) was used instead of undecafluorocyclohexylmethanol. Consequently, the targeted product (molecular weight: 518.36, yield 4.04 g, fractional yield 39.0%) was obtained.

### <Spectral data>

Nuclear magnetic resonance spectrum: (JNM-ECA500 manufactured by JEOL, Ltd. was used. Solvent: deuterochloroform)
¹H-NMR (500MHz): L56(dd,2H), 1.75(dd,4H), 1.89(s,3H), 2.10(dd,4H), 2.18(s,2H), 2.38(s,2H), 3.92(t,2H), 5.50(t,1H), 6.01(s,1H)
¹³C-NMR (125MEz): 18.30, 30.90, 34.88, 39.90, 40.00, 44.70, 76.00, 81.01, 124.83, 137.63, 166.39
¹⁹F-NMR (470MHz): -50.68(2F), -47.37(2F), -47.37(2F), -44.28(2F), -5.30(3F) GC-MS (EI): GCMS-QP2010 manufactured by Shimadzu Corp. was used. 518(M+, 5.5%), 432(43.2%), 390(14.4%), 377(42.5%), 364(17.2%), 219(12.4%), 133(10.8%), 121(4.9%), 105(6.3%), 92(100%), 77(11.3%), 69(57.7%), 55(6.7%)

### Example 5

### Synthesis of 3-[(perfluoropentyl)carbonyloxy]-1-adamantyl methacrylate represented by the following formula

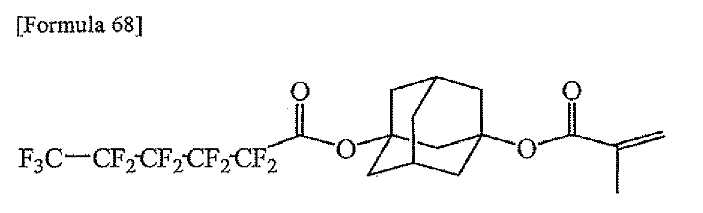

The reaction was carried out by a method similar to Example 1 except perfluorohexanoic acid (molecular weight: 314.05, 40 mmol, 12.56 g) was used instead of (undecafluorocyclohexyl)methanol. Consequently, the targeted product (molecular weight: 532.34, yield 5.48g, fractional yield 51.5%) was obtained.

### <Spectral data>

Nuclear magnetic resonance spectrum: JNM-ECA500 manufactured by JEOL, Ltd. was used. Solvent: deuterochloroform
¹H-NMR (500NMz): 1.63(br,2H), 1.90(s,3H), 2.11-2.22(m,8H), 2.44(s,2H), 2.58(s,2H), 5.52(s,1H), 6.03(s,1H)
¹³C-NMR (125MHz): 10.86, 23.85, 27.07, 32.16, 32.27, 37.43, 72.94, 79.72, 117.70, 129.96, 158.85
¹⁹F-NMR (465MHz): -126.17(2F), -122.58(d,4F), -118.39(2F), -75.73(3F) GC-MS (EI): GCMS-QP2010 manufactured by Shimadzu Corp. was used. 532(M+, 33.8%), 446(13.1%), 404(8.4%), 391(4.6%), 378(2.9%), 219(14.9%), 190(3.0%), 133(58.4%), 117(12.8%), 105(25.0%), 92(100%), 79(11.8%), 69(81.2%), 55(8.1%)

### Example 6

### Synthesis of 3-[(perfluoropentyl)carbonyloxy)oxy]-1-adamantyl methacrylate

Into a 200 mL flask equipped with an agitator, a thermometer and a Dean-Stark dehydrating apparatus with a condenser were charged adamantane-1,3-diol (molecular weight: 168.23, 30 mmol, 5.05 g, manufactured by Idemitsu Kosan Co., Ltd.), perfluorohexanoic acid (molecular weight: 314.(15, 33 mmol, 10.36 g), para-toluenesulfonic acid monohydrate (molecular weight: 190.22, 1.5 mmol, 0.29 g) and toluene (90 mL). Agitation of the mixture was initiated as well as the flask was immersed in an oil bath to raise the temperature to reflux, and water formed by the reaction with time was accumulated in the Dean-Stark dehydrating apparatus. After 4 hours, the reaction solution was analyzed by gas chromatography, confirming the disappearance of adamantan-1,3-diol. The reaction solution was cooled, then transferred to a separation funnel, and washed with 50 mL of a saturated aqueous sodium hydrogencarbonate solution, 50 mL of deionized water and 50 mL of saturated brine in this order. An organic layer was separated and dried after addition of magnesium sulfate. After filtration of magnesium sulfate, the solvent was evaporated to yield as a synthetic intermediate 3-hydroxy-1-adamantyl perfluorohexanoate (molecular weight: 464.27, yield 12.26 g, fractional yield 88.0%).
Into a 100 mL flask equipped with an agitator, a thermometer and a dropping funnel were charged 3-hydroxy-1-adamantyl perfluorohexanoate (20 mmol, 9.29 g) obtained as above, triethylamine (molecular weight: 101.19, 30 mmol, 3.04 g) and dry THF (50 mL). The flask was chilled on an ice bath to 0°C with stirring, to which methacryloyl chloride (molecular weight: 104.53, 24 mmol, 2.51 g) was gradually added using a dropping funnel. The temperature was raised to 60°C and after 5 hours the reaction solution was analyzed by gas chromatography, confirming disappearance of 3-hydroxy-1-adamantyl perfluorohexanoate. The reaction solution was cooled and then transferred to a separation funnel, to which 50 mL of hexane was added, followed by washing with 50 mL of a saturated aqueous sodium hydrogencarbonate solution, 50 mL of deionized water, and 50 mL of saturated brine in this order. An organic layer was separated and dried by addition of magnesium sulfate. After filtration of magnesium sulfate, the solvent was evaporated to yield the targeted product (yield 7.86g, fractional yield 73.8%).
Spectral data are identical with those of the compound obtained in Example 5.

### Industrial Applicability

Use of the polymerizable compound having the adamantane structure in the present invention and a resin composition thereof has in the field of photolithography the effect of preventing the liquid immersion medium from penetration and improving dry etching resistance in the liquid immersion exposure method as well as the effect of reducing adhesion to a mold and improving dry etching resistance in the nanoimprint method, thus allowing suitable application in the field of photolithography and nanoimprint lithography.

## Claims

1. A polymerizable compound having an adamantane structure with the structure represented by the general formula (1): 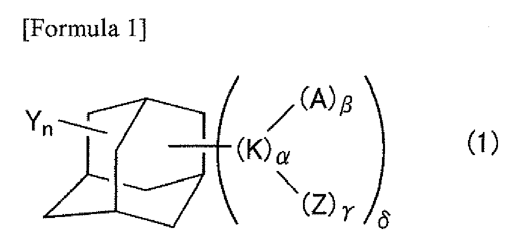 (wherein, A is the group containing a polymerizable group represented by the formulas (2) or (3) and a plurality of A may be identical or different, K is the linkage group represented by any one of the formulas (4) to (8) and a plurality of K may be identical or different, Z is the following fluorinated substituent and a plurality of Z may be identical or different, Y is a substituent on adamantane and represents a hydrogen atom, an alkyl group having 1 to 10 carbon atoms, a halogen atom, a hydroxyl, mercapto or methylcyano group or =O or =S formed by joining two Ys, a plurality of Y may be also identical or different, α is an integer of 1 or more, β and γ each are an integer of 0 or more, but one or more of A and Z are contained in the general formula (1), δ is an integer of 1 to 16, n is an integer of 0 to 15, and δ + n is equal to 16);
A (Polymerizable group):
[Formula 2] CH₂=CR^{o}- (2) CH≡C- (3)
wherein R⁰ represents a hydrogen atom, a fluorine atom, a methyl, ethyl or trifluoromethyl group;
K (Linkage group): 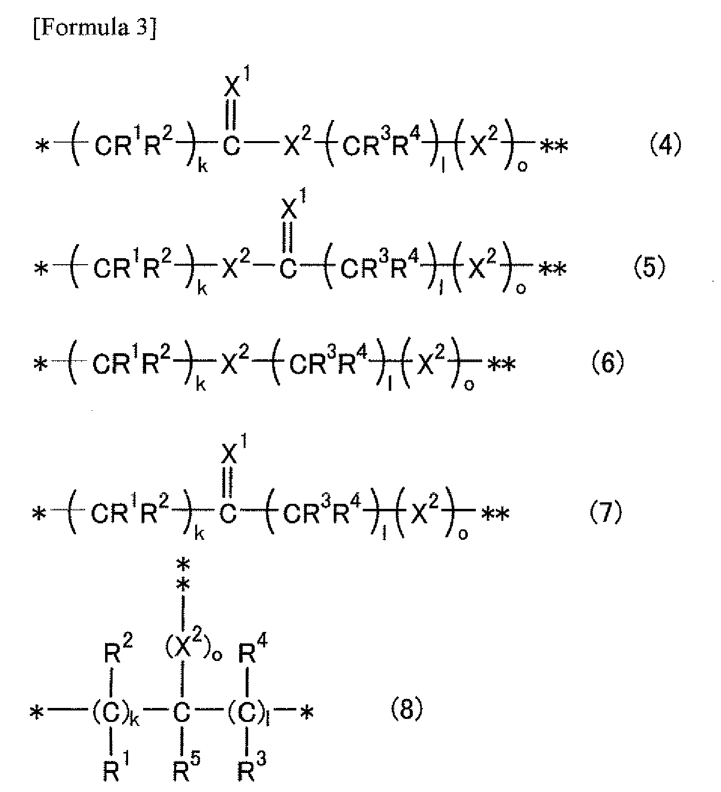 wherein R¹, R², R³, R⁴ and R⁵ each independently represent a hydrogen atom, an alkyl group having 1 to 10 carbon atoms optionally containing a heteroatom or a halogen atom, k and I each represent an integer of 0 to 10, X¹ and X² each independently represent an oxygen atom, a sulfur atom or an NR' group, R' is a hydrogen atom or an alkyl group having 1 to 10 carbon atoms optionally containing a heteroatom, single asterisk (*) represents the side of a polymerizable group or the side of an end group, a double asterisk (**) represents the side of an adamantane ring and o represents 0 or 1;
Z (Fluorinated substituent):
wherein Z represents an alkyl group having 1 to 30 carbon atoms or a cycloalkyl group having 5 to 30 carbon atoms, has to include a fluorine atom in part of the structure thereof, and optionally contains in part of the structure thereof at least one kind selected from a heteroatom or hydroxyl, mercapto, ether, thioether, cyano, ketone, thioketone, ketal, thioketal, acetal, thioacetal, lactone, thiolactone, carbonate, thiocarbonate, amine, amide, alkylsulfonyl, ester and thioester groups.)

2. The polymerizable compound having the adamantane structure according to claim 1, having a structure represented by the general formula (9): 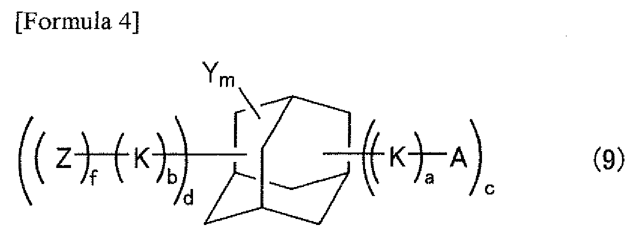 (wherein A (polymerizable group), K (linkage group), Z (fluorinated substituent), and Y (substituent on adamantane) are similar to those in the general formula (1), a, b, c, d and f each are an integer of 1 or more, m is an integer of 0 to 14, and c + d + m is equal to 16).

3. The polymerizable compound having the adamantane structure according to claim 2, having a structure represented by the general formula (10): 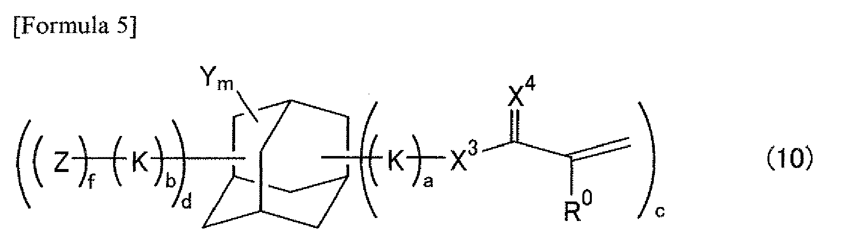 (wherein K (linkage group), Z (fluorinated substituent), and Y (substituent on adamantane) are similar to those in the general formula (1), R⁰ is similar to that in the general formula (2), a, b, c, d, f, and m are similar to those in the general formula (9), X³ and X⁴ each independently represent an oxygen atom, a sulfur atom or NR' group and R' is a hydrogen atom or an alkyl group having 1 to 10 carbon atoms optionally containing a heteroatom.)

4. The polymerizable compound having the adamantane structure according to claim 2, having a structure represented by the general formula (11): 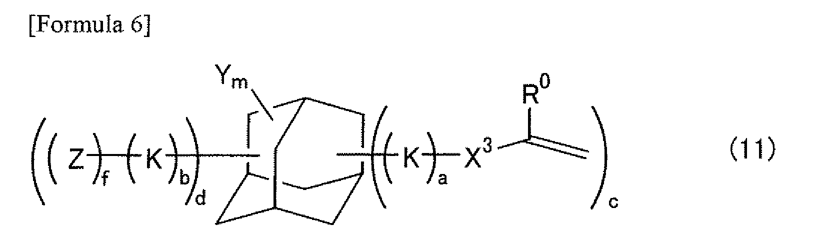 (wherein K (linkage group), Z (fluorinated substituent), Y (substituent on adamantane), R⁰, X³, a, b, c, d, f, and m are similar to those in the general formula (10).)

5. The polymerizable compound having the adamantane structure according to claim 3, having a structure represented by the general formula (12): 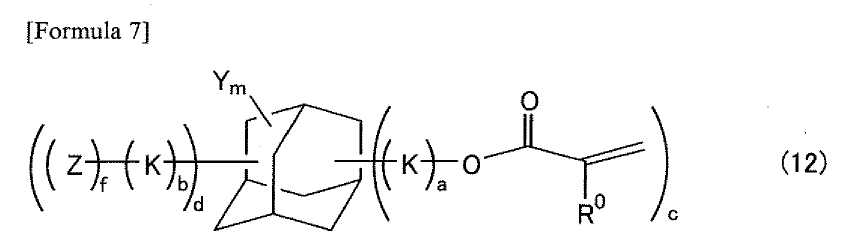 (wherein K (linkage group), Z (fluorinated substituent), Y (substituent on adamantane), R⁰, a, b, c, d, f, and m are similar to those in the general formula (10).)

6. The polymerizable compound having the adamantane structure according to claim 4, having a structure represented by the general formula (13): 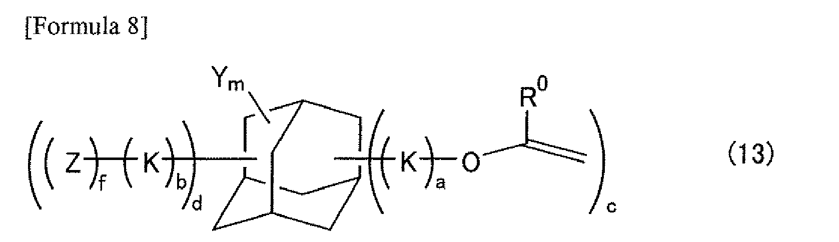 (wherein K (linkage group), Z (fluorinated substituent), Y (substituent on adamantane), R⁰, a, b, c, d, f, and m are similar to those in the general formula (10).)

7. The polymerizable compound having the adamantane structure according to claim 2, having a structure represented by the general formula (14): 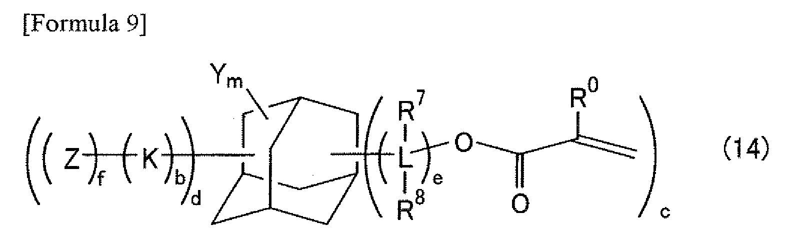 (wherein K (linkage group), Z (fluorinated substituent), Y (substituent on adamantane), R⁰, b, c, d, f, and m are similar to those in the general formula (10), R⁷ and R⁸ each independently represent a hydrogen atom, an alkyl group having 1 to 10 carbon atoms optionally containing a heteroatom or a halogen atom, L independently represents a carbon, oxygen, nitrogen or sulfur atom, a plurality of R⁷ and a plurality of R⁸ each may be identical or different, however, when L is an oxygen, nitrogen or sulfur atom, either one or both of R⁷ and R⁸ are absent, and e is an integer of 0 to 5.)

8. The polymerizable compound having the adamantane structure according to claim 2, having a structure represented by the general formula (15): 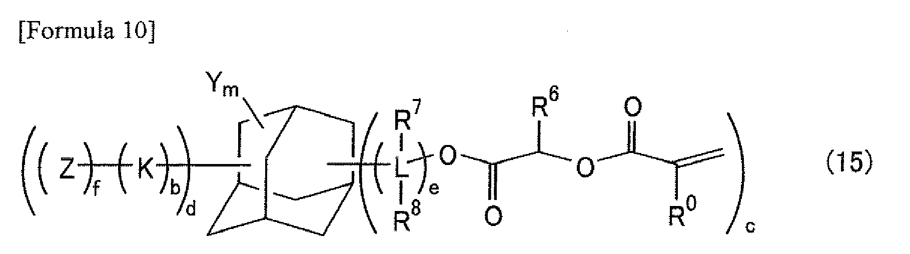 (wherein K (linkage group), Z (fluorinated substituent), Y (substituent on adamantane), L, R⁰, R⁷, R⁸, b, c, d, e, f, and m are similar to those in the general formula (14), and R⁶ represents a hydrogen atom, an alkyl group having 1 to 10 carbon atoms optionally containing a heteroatom or a halogen atom.)

9. The polymerizable compound having the adamantane structure according to claim 2, having a structure represented by the general formula (16): 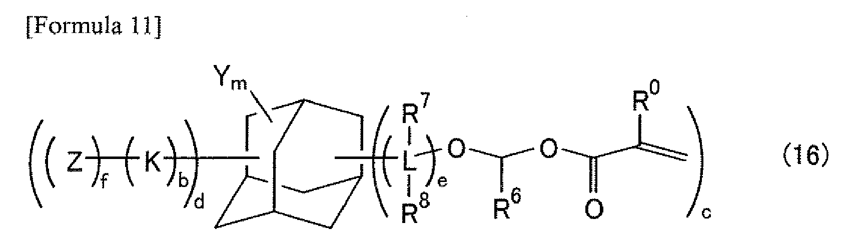 (wherein K (linkage group), Z (fluorinated substituent),Y (substituent on adamantane), L, R⁰, R⁶, R⁷, R⁸, b, c, d, e, f, and m are similar to those in the general formula (15).)

10. The polymerizable compound having the adamantane structure according to claim 2, having a structure represented by the general formula (17): 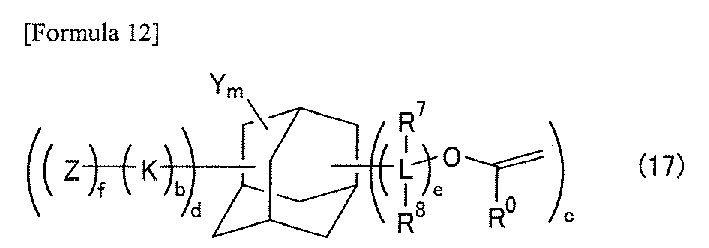 (wherein K (linkage group), Z (fluorinated substituent), Y (substituent on adamantane), L, R⁰, R⁷, R⁸, b, c, d, e, f, and m are similar to those in the general formula (15).)

11. The polymerizable compound having the adamantane structure according to claim 2, having a structure represented by the general formula (18): 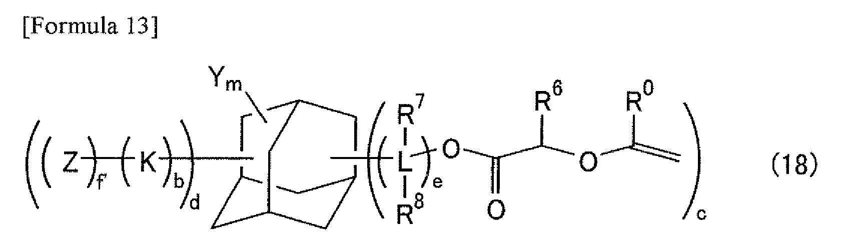 (wherein K (linkage group), Z (fluorinated substituent), Y (substituent on adamantane), L, R⁰, R⁶, R⁷, R⁸, b, c, d, e, f, and m are similar to those in the general formula (15).)

12. The polymerizable compound having the adamantane structure according to claim 2, having a structure represented by the general formula (19): 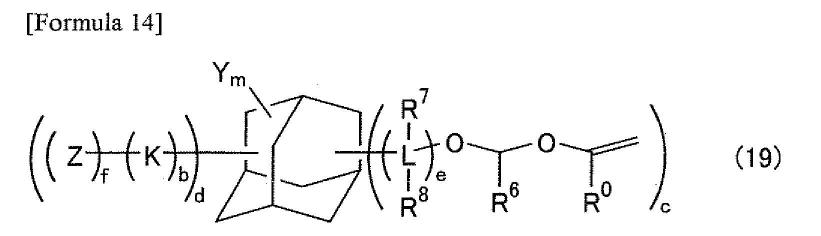 (wherein K (linkage group), Z (fluorinated substituent), Y (substituent on adamantane), L, R⁰, R⁶, R⁷, R⁸, b, c, d, e, f, and m are similar to those in the general formula (15).)

13. The polymerizable compound having the adamantane structure according to claim 2, in which at least one of R⁹ to R¹¹ in the general formula (20) is represented by any one of the general formula (21), the general formula (22), the general formula (23), the general formula (24), the general formula (25), and the general formula (2b): 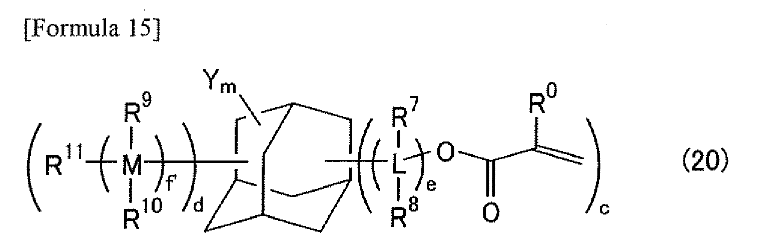 (wherein Y (substituent on adamantane), R⁰, c, d, and m are similar to those in the general formula (10), L and M each independently represent a carbon, oxygen, nitrogen or sulfur atom, R⁷, R⁸, R⁹, R¹⁰, and R¹¹ each independently represent a hydrogen atom, an alkyl group having 1 to 10 carbon atoms optionally containing a heteroatom, a halogen atom or =O or =S formed by joining two of R⁷, R⁸ and R⁹ to R¹¹, a plurality of R⁷, a plurality of R⁸, a plurality of R⁹, and a plurality of R¹⁰ and R¹¹ each may be identical or different, however, when L and M are an oxygen, nitrogen or sulfur atom, either one or both of R⁷ and R⁸ in L are absent and either one or both of R⁹ and R¹⁰ in M are absent, and e and f" each are an integer of 0 to 5); 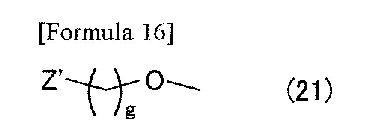 (wherein Z' (fluorinated substituent) represents an alkyl group having 1 to 30 carbon atoms or a cycloalkyl group having 5 to 30 carbon atoms, in which hydrogen atoms in the structure are fully fluorinated and optionally contain in part of the structure thereof at least one kind selected from a heteroatom, ether, thioether, cyano, ketone, thioketone, ketal, thioketal, acetal, thioacetal, lactone, thiolactone, carbonate, thiocarbonate, ester and thioester groups, and g is an integer of 0 or more); 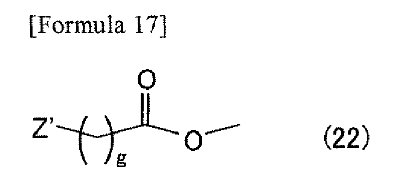 (wherein Z' (fluorinated substituent) and g are similar to those in the general formula (21)); 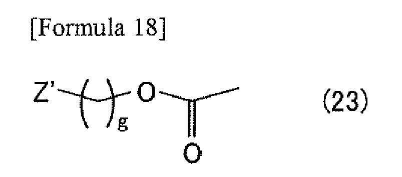 (wherein Z' (fluorinated substituent) and g are similar to those in the general formula (21)); 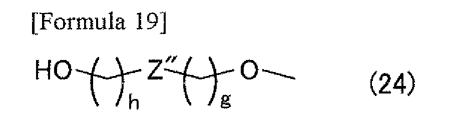 (wherein Z" (fluorinated substituent) represents an alkylene group having 1 to 30 carbon atoms or a cycloalkylene group having 5 to 30 carbon atoms, in which hydrogen atoms in the structure are fully fluorinated and optionally contain in part of the structure thereof at least one kind selected from a heteroatom, ether, thioether, cyano, ketone, thioketone, ketal, thioketal, acetal, thioacetal, lactone, thiolactone, carbonate, thiocarbonate, ester and thioester groups, and g and h are an integer of 0 or more); 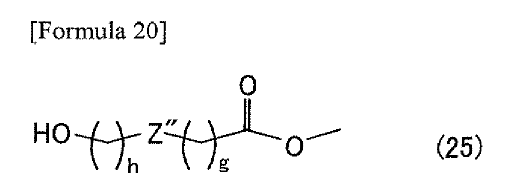 (wherein Z" (fluorinated substituent), g, and h are similar to those in the general formula (24)); and 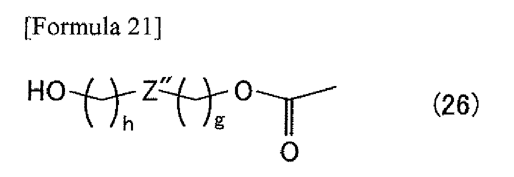 (wherein Z" (fluorinated substituent), g, and h are similar to those in the general formula (24).)

14. The polymerizable compound having the adamantane structure according to claim 2, in which at least one of R⁹ to R¹¹ in the general formula (27) is represented by any one of the general formula (28), the general formula (29), the general formula (30), the general formula (31), the general formula (32), and the general formula (33): 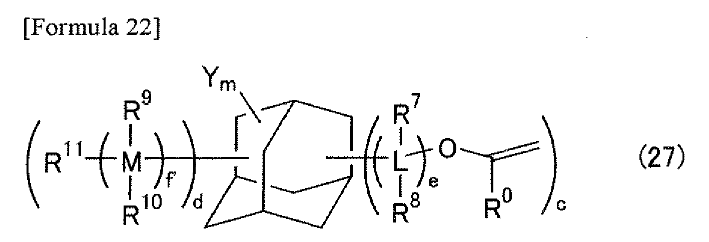 (wherein Y (substituent on adamantane), L, M, R⁰, R⁷, R⁸, R⁹, R¹⁰, R¹¹, c, d, e, and f" are similar to those in the general formula (20.); 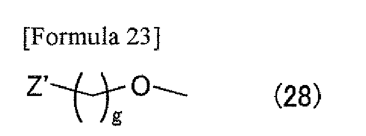 (wherein Z' (fluorinated substituent) and g are similar to those in the general formula (21)); 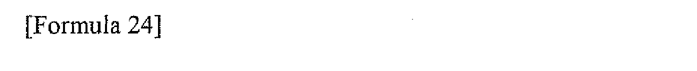 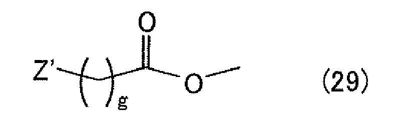 (wherein Z' (fluorinated substituent) and g are similar to those in the general formula (21)); 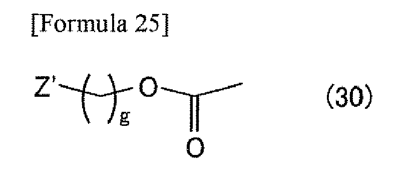 (wherein Z' (fluorinated substituent) and g are similar to those in the general formula (21)); 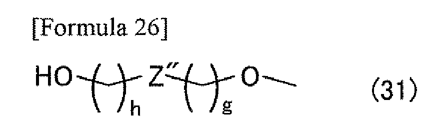 (wherein Z" (fluorinated substituent), g, and h are similar to those in the general formula (24)); 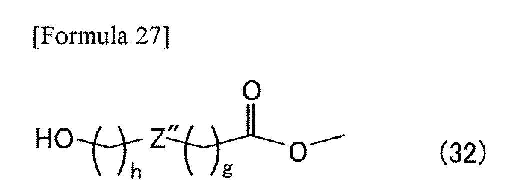 (wherein Z" (fluorinated substituent), g, and h are similar to those in the general formula (24); and 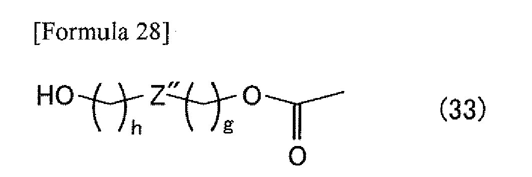 (wherein Z" (fluorinated substituent), g, and h are similar to those in the general formula (24).)

15. A production method of a polymerizable compound having the adamantane structure represented by the general formula (37), comprising reacting a compound of the general formula (34), in which at least one of R⁹, R¹⁰, and R¹² is represented by the general formula (35) with a compound represented by the general formula (36): 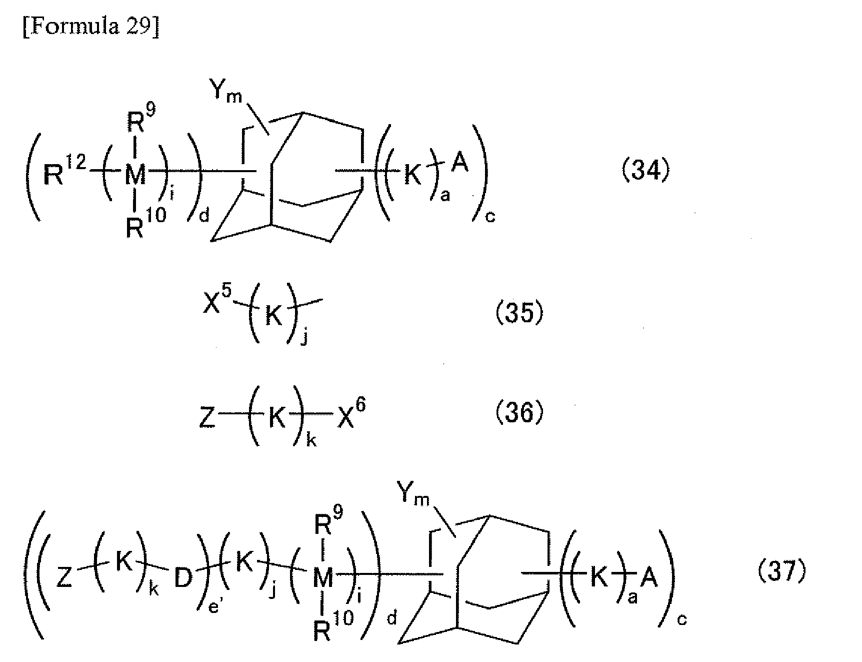 (wherein A (polymerizable group), K (linkage group), Z (fluorinated substituent), Y (substituent on adamantane), a, c, d, and m are similar to those in the general formula (9), e' is an integer of 1 or more, i is an integer of 0 to 5, j and k each are an integer of 0 or more, R⁹, R¹⁰, and R¹² each independently represent a hydrogen atom, an alkyl group having 1 to 10 carbon atoms optionally containing a heteroatom, a halogen atom or =O or =S formed by joining two of R⁹, R¹⁰ and R¹¹, a plurality of R⁹, a plurality of R¹⁰, a plurality of R¹² each may be identical or different, M independently represents a carbon, oxygen, nitrogen or sulfur atom, however, when M is an oxygen, nitrogen or sulfur atom, either one or both of R⁹ and R¹⁰ are absent, X⁵ and X⁶ are a reactive group and when X⁵ is selected from a hydrogen atom, a halogen atom, and an alkylsulfonyloxy, perfluoroalkylsulfonyloxy or alkyl-substituted phenylsulfonyloxy group, X⁶ represents a hydrogen atom or a group selected from a hydroxyl, mercapto, or amino group or salt thereof, whereas when X⁵ is selected from a hydrogen atom, a hydroxyl, mercapto or amino group or salt thereof, X⁶ represents a hydrogen atom, a halogen atom or a group selected from an akylsulfonyloxy, perfluoroalkylsulfonyloxy or alkyl-substituted phenylsulfonyloxy group, and D represents a linkage group formed by the reaction of X⁵ with X⁶.)

16. A production method of a polymerizable compound having the adamantane structure represented by the general formula (40), comprising reacting a compound represented by the general formula (38) with a compound represented by the general formula (39): 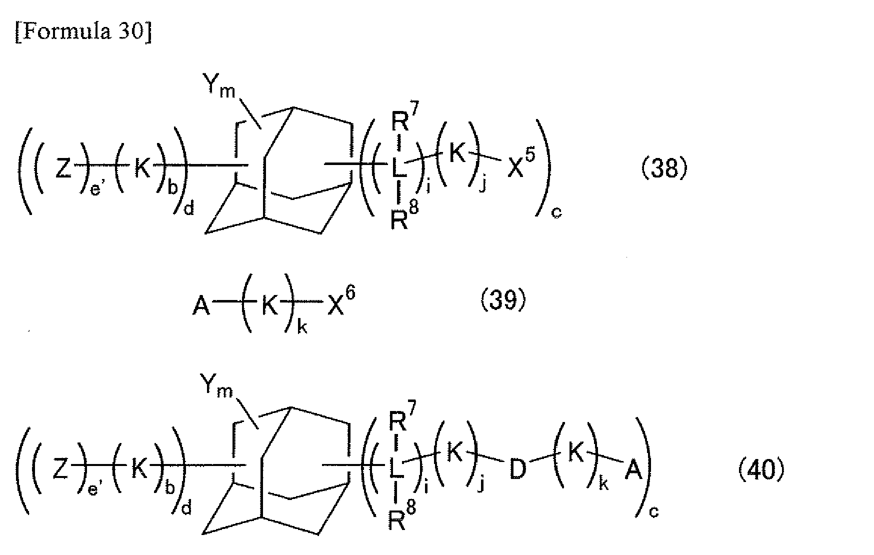 (wherein A (polymerizable group), K (linkage group), Z (fluorinated substituent)), Y (substituent on adamantane), X⁵, X⁶, D, c, d, e', i, j, k, and m are similar to those in the general formula (34) to (37) and L, R⁷, and R⁸ are similar to those in the general formula (14).)

17. A polymer comprising the polymerizable compound having the adamantane structure according to any of claims 1 to 14 as a constituent.

18. A photoresist composition comprising the polymer constituted of the polymerizable compound having the adamantane structure according to any of claims 1 to 14.

19. A thermocurable resin composition comprising the polymerizable compound having the adamantane structure according to any of claims 1 to 14 as a constituent.

20. A photocurable resin composition comprising the polymerizable compound having the adamantane structure according to any of claims 1 to 14 as a constituent.
